# EUROPEAN PATENT APPLICATION

(11) **EP 4 520 349 A1**
(43) Date of publication of application: **12.03.2025**
(21) Application number: 23830484.4
(22) Date of filing: 30.06.2023
(51) Int. Cl.: A61K 45/00, A61K 31/352, A61K 45/06, A61P 35/00

(54) **INTERFERING RNA FOR INHIBITING B7-H3 GENE EXPRESSION AND USE THEREOF**

(30) Priority: 30.06.2022 CN 202210760813
(71) Applicant: Jenkem Technology Co., Ltd. (Beijing), Haidian District Beijing 100192 (CN)
(72) Inventor: YANG, Mai, Beijing 100192 (CN); LIN, Meina, Beijing 100192 (CN); JIA, Hongli, Beijing 100192 (CN); WANG, Qingbin, Beijing 100192 (CN); CHEN, Xiaomeng, Beijing 100192 (CN); ZHAO, Xuan, Beijing 100192 (CN)
(74) Representative: HGF
(86) International application number: PCT/CN2023/104455
(87) International publication number: WO 2024/002320

(57) **Abstract**

The present invention provides an interfering RNA for inhibiting B7-H3 expression, use thereof, and a method for preparing cells with reduced B7-H3 expression. The interfering RNA is siRNA, a target sequence of which comprises nucleotide sequences set forth in SEQ ID NOs: 1-25. The interfering RNA can effectively inhibit B7-H3 gene expression, thus being useful for preventing or treating B7-H3 related diseases.

## Description

### TECHNICAL FIELD

The present invention relates to the technical field of molecular biology and biomedicine, and in particular to an interfering RNA, particularly siRNA, for inhibiting B7-H3 expression and use thereof.

### BACKGROUND

T cell-mediated cellular immunity plays an important role in anticancer processes. T cell activation depends on two signals. The first signal arises from the specific recognition of the major histocompatibility complex (MHC) on the surface of antigen-presenting cells (APCs) by the T cell receptor (TCR). The second signal is provide by co-stimulatory molecules (such as members of the B7 family) or other molecules on the surface of APCs. In addition to co-stimulatory molecules, co-inhibitory molecules are also present on the surface of APCs, which act synergistically to inhibit T cell activity and prevent over-activation. The balance between co-stimulatory and co-inhibitory molecules determines the function and tolerance of T cells (Nurieva R, EMBO J, 2006; Ni L, Immunol Rev, 2017), these molecules are also referred to as immune checkpoints. Immune checkpoints are expressed not only on the surface of APCs but also on tumor cells. Through co-inhibitory molecules on their surface, tumor cells interact with corresponding receptors on T cells, suppressing the immune response of the T cells and thereby enabling immune escape.

B7-H3 (CD276, Gene ID: 80381) belongs to the B7 family and is located on human chromosome 15, with four known transcripts. B7-H3 is a type I transmembrane protein located on the cell membrane and exists in two subtypes, 4IgB7-H3 and 2IgB7-H3. Early studies have found that B7-H3 acts as co-stimulatory molecule, capable of inducing T cell immune responses (Chapoval AI, Nat Immunol, 2001). However, increasing evidence from subsequent studies has shown that B7-H3 primarily functions as a co-inhibitory molecule, helping tumor cells to achieve immune escape. Recent studies have demonstrated that B7-H3 promotes tumor cell behaviors, including proliferation, invasion, and metastasis, in various cancers such as head and neck squamous cell carcinoma and prostate cancer. In addition, studies have also shown that B7-H3 enhances tumor cell resistance to radiopharmaceuticals and chemotherapeutic drugs (Zhang W, Int J Oncol, 2015; Wang C, Cell Stem Cell, 2021). The B7-H3 protein is expressed in variety of tumors, such as gastric cancer, lung cancer, prostate cancer, renal cancer, ovarian cancer, endometrial cancer, colorectal cancer, pancreatic cancer, liver cancer, oral cancer, bladder cancer, breast cancer, etc. (Ni L, Immunol Rev. 2017; Kontos F, Clin Cancer Res, 2021), while its expression in normal tissues is restricted (Yuan H, J Urol, 2011). This restricted expression profile makes it possible to treat cancer by inhibiting B7-H3 expression.

Patent CN100574803 discloses an antibody therapy for the treatment of neoplastic diseases using immunomodulatory antibodies, preferably anti-B7 antibodies and anti-CD20 antibodies, to trigger human antibody effector mechanisms and cause apoptosis or death of target cells. Patent CN113402619B discloses a B7-H3-targeted fully human chimeric antigen receptor co-expressing IL-21, iNKT cells, and use thereof. The chimeric antigen receptor comprises a B7-H3 antigen binding domain, a transmembrane domain, an intracellular signal domain, and IL-21. The B7-H3.CAR/IL-21-iNKT cells targeting B7-H3, which are prepared through experimental verification, have strong cytokine release capacity and tumor cell killing capacity, and can effectively eliminate tumor cells.

Currently, drugs that inhibit immune checkpoints are parimarily antibody-based drugs, however, the development of antibody drugs is limited by the spatial structure of protein molecules, such that it is difficult to prepare certain targets into drugs. Interfering RNA (e.g., siRNA) is a short RNA segment of about 20 nucleotides and is also one of the components of RNA-induced silencing complexes (RISCs). Interfering RNA helps RISCs to specifically target mRNA and induces the silencing or degradation of mRNA based on the principle of complementary base pairing. Compared to the limitations of antibody drugs, interfering RNA theoretically allows for the silencing of any gene. Therefore, interfering RNA is used to silence B7-H3 in the present application.

### SUMMARY

The present invention overcomes the defects in the prior art, and provides an interfering RNA that can specifically target and silence B7-H3 mRNA, and reduce B7-H3 expression, thus reducing the inhibition effect of tumor cells on T cells and enhancing the immune response. Therefore, the interfering RNA is used in treating solid tumors (e.g., squamous cell carcinoma of the head and neck and prostate cancer).

In a first aspect of the present invention, provided is an interfering RNA targeting B7-H3.

Specifically, the interfering RNA inhibits the expression of B7-H3 mRNA.

Specifically, the interfering RNA described above comprises a sense strand and an antisense strand complementary to and paring to the sense strand reversely.

Specifically, the sense strand described above comprises a nucleotide sequence set forth in any one or two or more of SEQ ID NOs: 26, 28, 30, 32, 34, 36, 38, 40, 42, 44, 46, 48, 50, 52, 54, 56, 58, 60, 62, 64, 66, 68, 70, 72, and 74. Specifically, the antisense strand described above comprises a nucleotide sequence set forth in any one or two or more of SEQ ID NOs: 27, 29, 31, 33, 35, 37, 39, 41, 43, 45, 47, 49, 51, 53, 57, 59, 61, 63, 65, 67, 69, 71, 73, and 75.

Specifically, the interfering RNA described above is selected from siRNA, dsRNA, shRNA, aiRNA, miRNA, and combinations thereof.

In one embodiment of the present invention, the interfering RNA described above is siRNA. Specifically, a piece of double-stranded RNA with a length of 19-23 nt, after entering a cell, is processed by a series of enzymes, and forms a RISC complex with other proteins, specifically targeting and silencing a target gene by means of complementary base pairing.

In one embodiment of the present invention, the interfering RNA described above is chemically synthesized. Specifically, the target sequence of the interfering RNA described above comprises a nucleotide sequence set forth in any one or two or more (e.g., 1, 2, 3, 4, 5, 6, 7, 8, 9, 10, 11, 12, 13, 14, 15, 16, 17, 18, 19, 20, 21, 22, 23, 24, and 25) of SEQ ID NOs: 1-25.

Specifically, the target sequence of the interfering RNA described above consists of the nucleotide sequences set forth in any one or two or more (e.g., 1, 2, 3, 4, 5, 6, 7, 8, 9, 10, 11, 12, 13, 14, 15, 16, 17, 18, 19, 20, 21, 22, 23, 24, and 25) of SEQ ID NOs: 1-25.

Specifically, the interfering RNA further comprises an over-hang, and preferably, the interfering RNA comprises 1-10 over-hangs.

Specifically, the over-hang is located at the 3' terminus of a sense strand and/or an antisense strand of the interfering RNA.

In one embodiment of the present invention, the over-hang is a deoxynucleoside.

In one embodiment of the present invention, the over-hang is dTdT, dTdC, or dUdU.

In some embodiments, the interfering RNA described above further comprises at least one modification, and a modified interfering RNA has better properties, such as higher stability and lower immune stimulation, than a corresponding unmodified interfering RNA.

Specifically, the modification comprises modifications on chemical structures of a base, a sugar ring, and/or a phosphate.

Specifically, the modifications on a base comprise, but are not limited to, pyrimidine modifications at site 5, purine modifications at site 8, and/or 5-bromouracil substitutions.

Specifically, the modifications on a sugar ring comprise, but are not limited to, substitutions of 2'-OH with H, OZ, Z, halo, SH, SZ, NH2, NHZ, NZ2, CN, or other groups, Z being an alkyl group. Preferably, the modification further comprises nucleotides having inosine, queuosine, xanthine, 2'-methylribose, non-natural phosphodiester bond (e.g., methylphosphonate and phosphorothioate), and/or peptide.

In some embodiments, the interfering RNA molecule described above is a modified interfering RNA comprising a nucleotide sequence set forth in any one or two or more of SEQ ID NOs: 80-91.

In some embodiments, the modified interfering RNA described above comprises a sense strand and an antisense strand complementary to and paring to the sense strand reversely.

In some embodiments, in the modified interfering RNA described above, the sense strand comprises a nucleotide sequence set forth in SEQ ID NO: 80, and the antisense strand comprises a nucleotide sequence set forth in SEQ ID NO: 81.

In some embodiments, in the modified interfering RNA described above, the sense strand comprises a nucleotide sequence set forth in SEQ ID NO: 82, and the antisense strand comprises a nucleotide sequence set forth in SEQ ID NO: 83.

In some embodiments, in the modified interfering RNA described above, the sense strand comprises a nucleotide sequence set forth in SEQ ID NO: 84, and the antisense strand comprises a nucleotide sequence set forth in SEQ ID NO: 85.

In some embodiments, in the modified interfering RNA described above, the sense strand comprises a nucleotide sequence set forth in SEQ ID NO: 86, and the antisense strand comprises a nucleotide sequence set forth in SEQ ID NO: 87.

In some embodiments, in the modified interfering RNA described above, the sense strand comprises a nucleotide sequence set forth in SEQ ID NO: 88, and the antisense strand comprises a nucleotide sequence set forth in SEQ ID NO: 89.

In some embodiments, in the modified interfering RNA described above, the sense strand comprises a nucleotide sequence set forth in SEQ ID NO: 90, and the antisense strand comprises a nucleotide sequence set forth in SEQ ID NO: 91.

In a second aspect of the present invention, provided is a delivery system comprising the interfering RNA according to the first aspect and a vector.

Specifically, the vector is a viral vector or a non-viral vector.

Specifically, the viral vector comprises: one or a combination of two or more of lentivirus vector, retrovirus vector, adenovirus vector, adeno-associated virus vector, poxvirus vector, or herpesvirus vector. Specifically, the non-viral vector comprises: any one or a combination of two or more of liposome, lipid nanoparticle, polymer, polypeptide, antibody, and aptamer.

Specifically, the lipid nanoparticle/liposome comprises: one or a combination of two or more of cationic lipid, neutral lipid, polyethylene glycol lipid, phospholipid, sterol, and anionic lipid.

Specifically, the cationic lipid comprises: one or a combination of two or more of stearamide (SA), lauryltrimethylammonium bromide, hexadecyltrimethylammonium bromide, myristyltrimethylammonium bromide, dimethyldioctadecylammonium bromide (DDAB), ((4-hydroxybutyl)azadialkyl)bis(hexane-6,1-diyl)bis(2-hexyldecanoate) (ALC-0315), 1,2-dioleoyloxy-3-(trimethylammonium)propane (DOTAP), 1,2-di-(9Z-octadecenoyl)-3-trimethylammonium-propane and 1,2-dihexadecanoyl-3-trimethylammonium-propane, 3β-[N-(N',N'-dimethylaminoethane)-carbamoyl]cholesterol (DC-cholesterol), dimethyldioctadecylammonium (DDA), 1,2-dimyristoyl-3-trimethylammonium propane (DMTAP), dipalmitoyl(C16:0)trimethylammonium propane (DPTAP), distearoyltrimethylammonium propane (DSTAP), N-[1-(2,3-diallyloxy)propyl]-N,N,N-trimethylammonium chloride (DOTMA), N,N-dioleoyl-N,N-dimethylammonium chloride (DODAC), 1,2-dioleoyl-sn-trioxy-3-ethylphosphocholine (DOEPC), 1,2-dioleoyl-3-dimethylammonium propane (DODAP), 1,2-dioleyloxy-3-dimethylaminopropane (DLinDMA), 1,2-ditetradecanoyl-3-dimethylammonium-propane, 1,2-dihexadecanoyl-3-dimethylammonium-propane and 1,2-dioctadecyl-3-dimethylammonium-propane, 1,2-dioleoyl-c-(4'-trimethylammonium)-butyryl-sn-glycerol (DOTB), dioctadecylamidoalanyl spermine, SAINT-2, polycationic lipid 2,3-dioleoyloxy-N-[2(spermine-carboxamido)ethyl]-N,N-dimethyl-1-propanaminium trifluoroacetate (DOSPA), 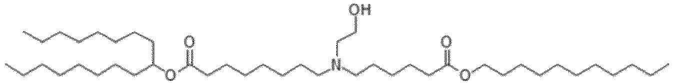 (SM-102), 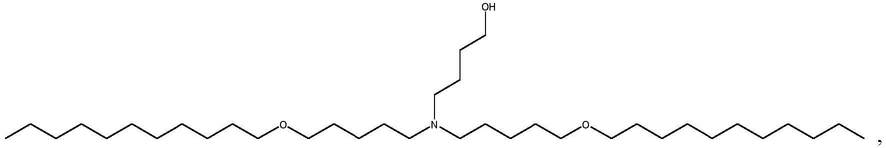 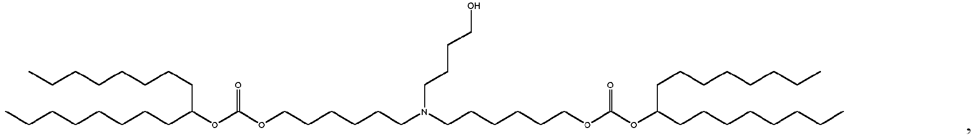 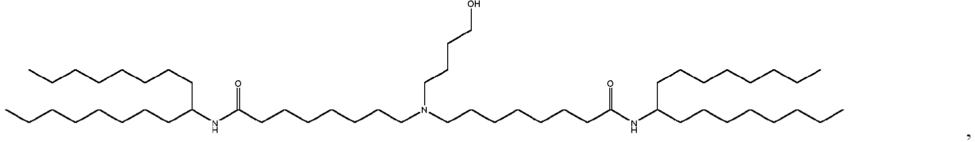 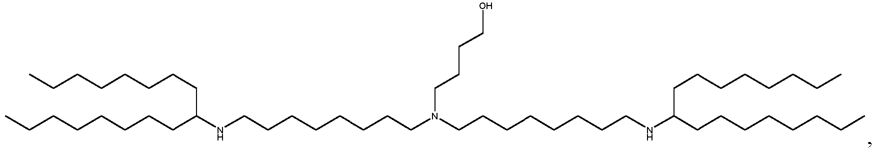 etc.

Specifically, the neutral lipid comprises: one or a combination of two or more of 1,2-distearoyl-sn-glycerol-3-phosphocholine (DSPC), 1,2-dipalmitoyl-sn-glycerol-3-phosphocholine (DPPC), 1,2-dioleoyl-sn-glycerol-3-phosphoethanolamine (DOPE), 1,2-dipalmitoyl-sn-glycerol-3-phosphoethanolamine (DPPE), 1,2-dimyristoyl-sn-glycero-3-phosphoethanolamine (DMPE), 2-dioleoyl-sn-glycero-3-phospho-(1'-rac-glycerol) (DOPG), oleoyl phosphatidylcholine (POPC), 1-palmitoyl-2-oleoyl phosphatidylethanolamine (POPE), 1,2-distearoyl-sn-glycero-3-phosphoethanolamine (DSPE), cholesterol, etc.

Specifically, the polyethylene glycol lipid comprises: one or a combination of two or more of 2-[(polyethylene glycol)-2000]-N,N-ditetradecylacetamide (ALC-0159), 1,2-dimyristoyl-sn-glyceromethoxypolyethylene glycol (PEG-DMG), **1,2-distearyl-sn-glycero-3-phosphoethanolamine-N-[amino(polyethylene** glycol)] (PEG-DSPE), PEG-disterol glycerol (PEG-DSG), PEG-dipalmitoyl, PEG-dioleyl, PEG-distearyl, PEG-diacylglycerol amide (PEG-DAG), PEG-dipalmitoyl phosphatidylethanolamine (PEG-DPPE) or PEG-1,2-dimyristoylacyloxypropyl-3-amine (PEG-c-DMA), 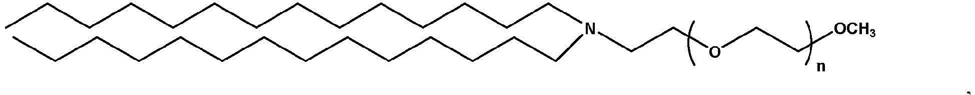 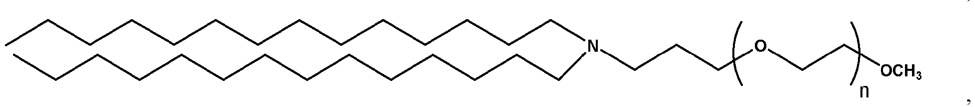 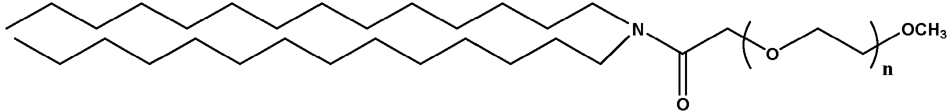 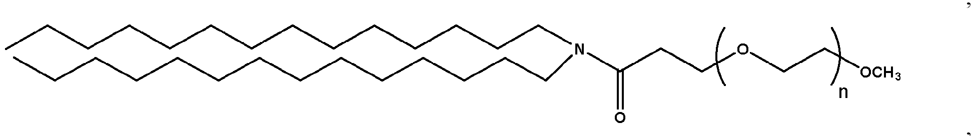 etc.,
wherein n is an integer selected from 20-300.

Specifically, the polyethylene glycol lipid is polyethylene glycol lipid with a single molecular weight, and preferably the polyethylene glycol lipid comprises: 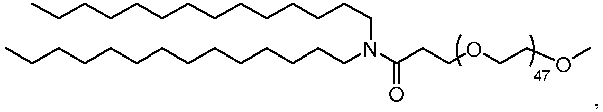 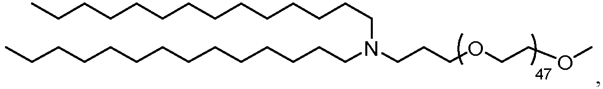 and

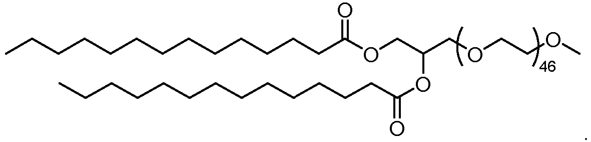

Specifically, the cationic lipid is a steroid-cationic lipid compound:

The structure of the compound is: 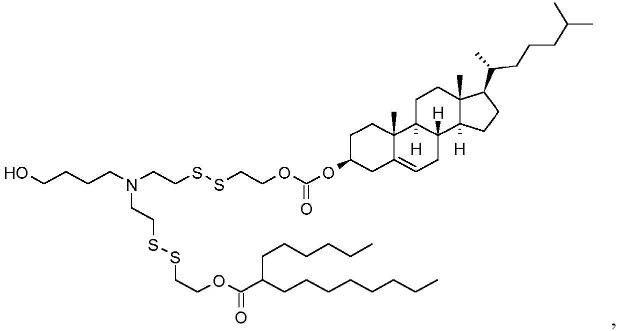 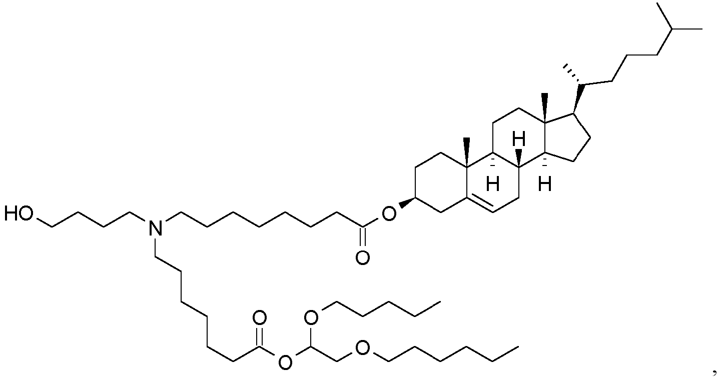 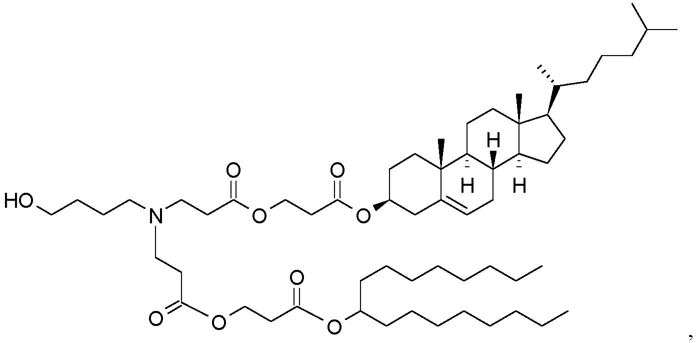 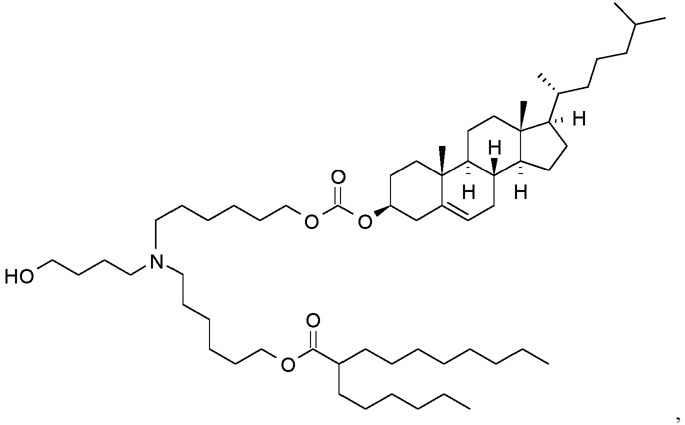 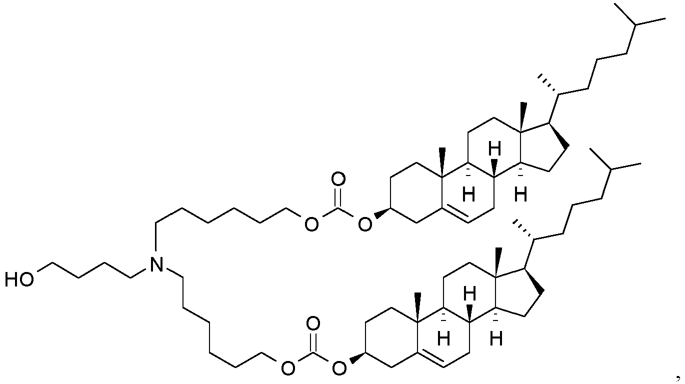 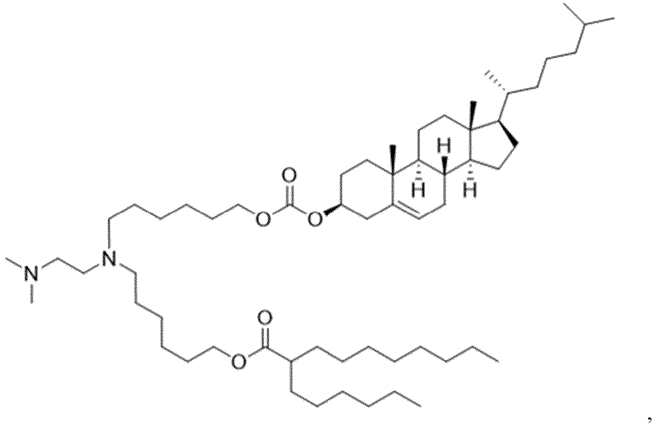 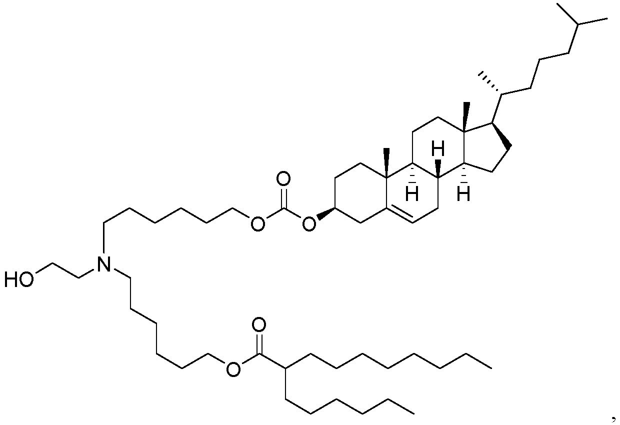 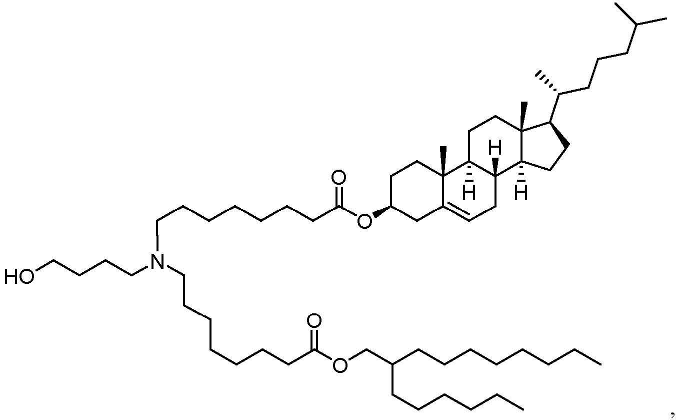 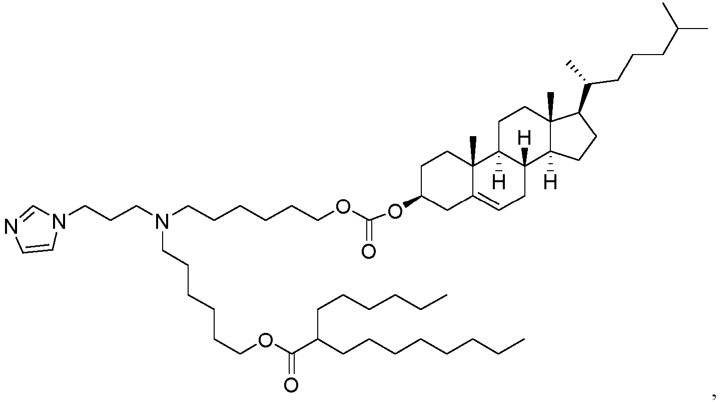 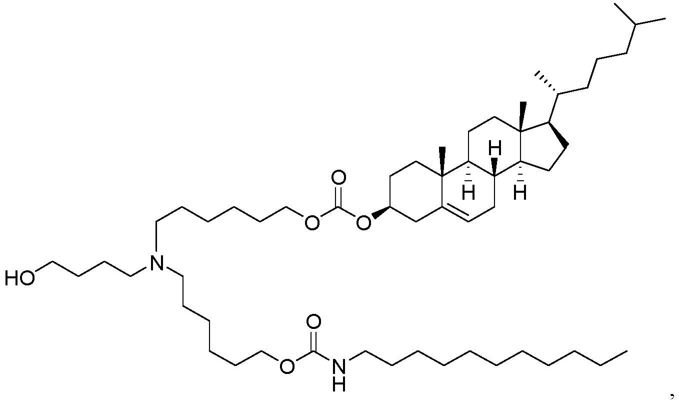 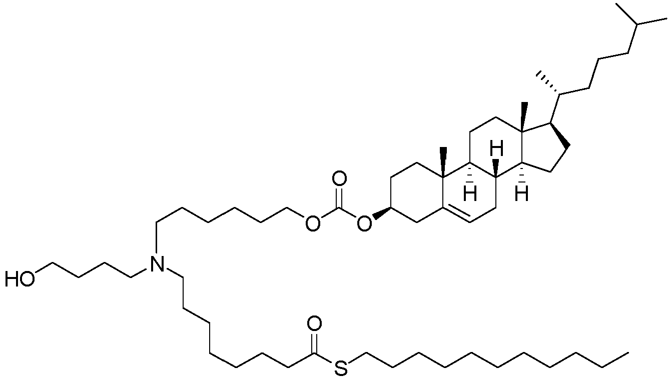 , or

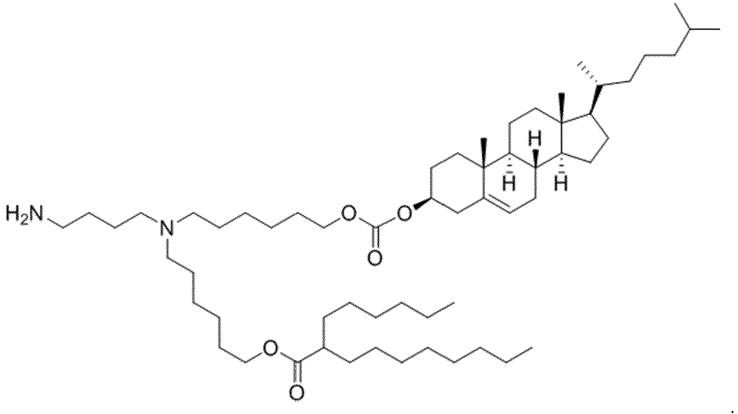

Specifically, the anionic lipid comprises: one or a combination of two or more of dioleoyl phosphatidylglycerol, dioleoyl phosphatidylethanolamine, etc.

Specifically, the sterol comprises: one or a combination of two or more of avenasterol, β-sitosterol, brassicasterol, ergocalciferol, campesterol, cholestanol, cholesterol, coprosterol, dehydrocholesterol, desmosterol, dihydroergocalciferol, dihydrocholesterol, dihydroergosterol, campesterol, epicholesterol, ergosterol, fucosterol, hexahydrolumisterol, hydroxycholesterol, lanosterol, lumisterol, saringosterol, sitostanol, sitosterol, stigmastanol, stigmasterol, cholic acid, glycocholic acid, taurocholic acid, deoxycholic acid, and lithocholic acid.

Specifically, the vector described above can be any vector suitable for delivering the interfering RNA of the present invention described above to a target tissue or a target cell, such as those disclosed in the prior art (e.g., Chen Zhonghua, Zhu Desheng, Li Jun, Huang Zhanqin, "Advances in non-viral vector research of siRNA". Chinese Pharmacological Bulletin. 2015, 31 (7): 910-4; Wang Rui, Qu Bingnan, Yang Jing. "Advances in research of siRNA-carrying nano preparation". China Pharmacy 2017, 28 (31): 4452-4455).

In a third aspect of the present invention, provided is a preparation method for a cell with reduced B7-H3 expression, and the preparation method comprises introducing the interfering RNA according to the first aspect or the delivery system according to the second aspect into cells.

Specifically, the cells described above are immune cells or tumor cells, and the immune cells are preferably lymphocytes, dendritic cells, monocytes, macrophages, granulocytes or mast cells, and more preferably T cells; the tumor cells are preferably head and neck cancer cells, prostate cancer cells, lung cancer cells, liver cancer cells, esophageal cancer cells, leukemia cells, cervical cancer cells, colorectal cancer cells, pancreatic cancer cells, renal cancer cells, bladder cancer cells, breast cancer cells, gastric cancer cells, oral cancer cells, ovarian cancer cells, osteosarcoma cells, brain tumor cells, or glioblastoma cells.

In a fourth aspect of the present invention, provided is a cell comprising the interfering RNA according to the first aspect or the delivery system according to the second aspect.

Specifically, the cells described above are immune cells or tumor cells, and the immune cells are preferably lymphocytes, dendritic cells, monocytes, macrophages, granulocytes or mast cells, and more preferably T cells; the tumor cells are preferably head and neck cancer cells, prostate cancer cells, lung cancer cells, liver cancer cells, esophageal cancer cells, leukemia cells, cervical cancer cells, colorectal cancer cells, pancreatic cancer cells, renal cancer cells, bladder cancer cells, breast cancer cells, gastric cancer cells, oral cancer cells, ovarian cancer cells, osteosarcoma cells, brain tumor cells, or glioblastoma cells.

In a fifth aspect of the present invention, provided are a pharmaceutical composition comprising the cell according to the fourth aspect, the interfering RNA according to the first aspect or the delivery system according to the second aspect, and a pharmaceutically acceptable excipient.

In a sixth aspect of the present invention, provided is use of the cell according to the fourth aspect, the interfering RNA according to the first aspect, or the delivery system according to the second aspect in preparing a medicament for preventing and/or treating a disease associated with B7-H3.

Specifically, the disease described above includes, but is not limited to, cancer, inflammation, malignant hematologic disorders, and the like, in particular, cancer.

Specifically, the cancer described above includes, but is not limited to, head and neck cancer (squamous cell carcinoma of the head and neck), prostate cancer, lung cancer (e.g., non-small cell lung cancer), liver cancer, esophageal cancer, leukemia (myeloid leukemia), cervical cancer, colorectal cancer, pancreatic cancer, renal cancer, bladder cancer, breast cancer, gastric cancer, oral cancer (oral epithelial cancer), ovarian cancer, osteosarcoma, brain tumor, glioblastoma, and the like.

Specifically, the inflammation described above includes, but is not limited to, rheumatoid arthritis, meningitis (bacterial meningitis), pancreatitis (acute pancreatitis), pneumonitis, and prostatitis.

In a seventh aspect of the present invention, provided is use of the cell according to the fourth aspect, the interfering RNA according to the first aspect, or the delivery system according to the second aspect in designing a medicament for preventing and/or treating a disease associated with B7-H3.

Specifically, the disease described above includes, but is not limited to, cancer, inflammation, malignant hematologic disorders, and the like, in particular, cancer.

Specifically, the cancer described above includes, but is not limited to, head and neck cancer (squamous cell carcinoma of the head and neck), prostate cancer, lung cancer (e.g., non-small cell lung cancer), liver cancer, esophageal cancer, leukemia (myeloid leukemia), cervical cancer, colorectal cancer, pancreatic cancer, renal cancer, bladder cancer, breast cancer, gastric cancer, oral cancer (oral epithelial cancer), ovarian cancer, osteosarcoma, brain tumor, glioblastoma, and the like.

Specifically, the inflammation described above includes, but is not limited to, rheumatoid arthritis, meningitis (bacterial meningitis), pancreatitis (acute pancreatitis), pneumonitis, and prostatitis.

In an eighth aspect of the present invention, provided is use of the cell according to the fourth aspect, the interfering RNA according to the first aspect, the delivery system according to the second aspect, or the pharmaceutical composition according to the fifth aspect in inhibiting B7-H3 gene expression in living cells.

In a ninth aspect of the present invention, provided is a method for inhibiting B7-H3 gene expression in a subject in need thereof, comprising a step of administering to the subject a therapeutically effective amount of the interfering RNA of the present invention described above and/or a vector comprising the interfering RNA described above and/or a cell with reduced B7-H3 expression and/or the pharmaceutical composition of the present invention. In a tenth aspect of the present invention, provided is a method for preventing and/or treating a disease associated with B7-H3, comprising a step of administering to a subject a therapeutically effective amount of the interfering RNA of the present invention described above and/or a vector comprising the interfering RNA described above and/or a cell with reduced B7-H3 expression and/or the pharmaceutical composition of the present invention.

Specifically, the disease described above includes, but is not limited to, cancer, inflammation, malignant hematologic disorders, and the like, in particular, cancer.

Specifically, the cancer described above includes, but is not limited to, head and neck cancer (squamous cell carcinoma of the head and neck), prostate cancer, lung cancer (e.g., non-small cell lung cancer), liver cancer, esophageal cancer, leukemia (myeloid leukemia), cervical cancer, colorectal cancer, pancreatic cancer, renal cancer, bladder cancer, breast cancer, gastric cancer, oral cancer (oral epithelial cancer), ovarian cancer, osteosarcoma, brain tumor, glioblastoma, and the like.

Specifically, the inflammation described above includes, but is not limited to, rheumatoid arthritis, meningitis (bacterial meningitis), pancreatitis (acute pancreatitis), pneumonitis, and prostatitis.

In an eleventh aspect of the present invention, provided is a medicament or kit comprising the interfering RNA according to the first aspect, the delivery system according to the second aspect, or the cell according to the fourth aspect.

In a twelfth aspect of the present invention, provided is an antibody-nucleic acid drug conjugate comprising the interfering RNA according to the first aspect or the delivery system according to the second aspect.

Specifically, the antibody-nucleic acid drug conjugate has a general formula (I): 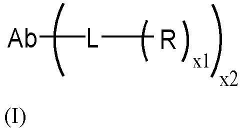
wherein R is the interfering RNA according to any one of claims 1-8;
x1 is an integer of 1-144; preferably, x1 is an integer of 1-9; more preferably, x1 is an integer of 1-3;
x2 is an integer of 1-8; preferably, x2 is an integer of 1-2;
Ab is an antibody, a protein, a polypeptide, or an oligonucleotide; and
L is a linking unit connecting Ab and R.

Specifically, the Ab is selected from a monoclonal antibody and a polyclonal antibody; preferably, Ab is a monoclonal antibody.

Specifically, the Ab is selected from: an anti-HER2 antibody, an anti-EGFR antibody, an anti-PMSA antibody, an anti-VEGFR antibody, an anti-CD30 antibody, an anti-CD22 antibody, an anti-CD56 antibody, an anti-CD29 antibody, an anti-GPNMB antibody, an anti-CD138 antibody, an anti-CD74 antibody, an anti-ENPP3 antibody, an anti-Nectin-4 antibody, an anti-EGFRVIII antibody, an anti-SLC44A4 antibody, an anti-mesothelin antibody, an anti-ET8R antibody, an anti-CD37 antibody, an anti-CEACAM5 antibody, an anti-CD70 antibody, an anti-MUC16 antibody, an anti-CD79b antibody, an anti-MUC16 antibody, and an anti-MUC1 antibody.

In some embodiments of the present invention, the antibody-nucleic acid drug conjugate has the following structure: 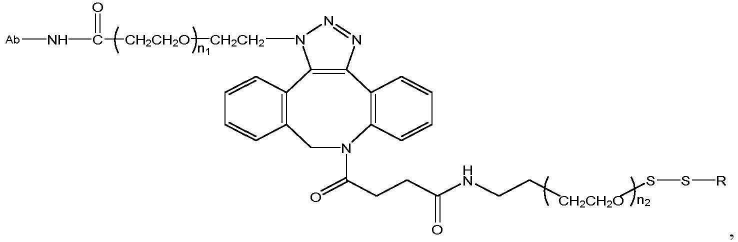 wherein n1 and n2 are independently selected from integers of 4-100, preferably integers of 4-24.

The inventors of the present invention obtain an interfering RNA (e.g., siRNA) via design and synthesis, which can effectively inhibit B7-H3 gene expression and provides a new option for preventing or treating a disease associated with B7-H3 (particularly, cancers associated with B7-H3).

The term "interfering RNA" includes a single strand RNA (e.g., mature miRNA, ssRNA oligonucleotide, or ssDNA oligonucleotide) or a double strand RNA (i.e., siRNA, dsRNA, shRNA, aiRNA, or pre-miRNA) which is capable of reducing or inhibiting expression of a target gene or sequence (e.g., by mediating degradation and/or inhibiting translation of mRNA complementary to the interfering RNA in sequence) when the interfering RNA and the target gene or sequence are in the same cell.

The siRNA is a small interfering RNA, and each strand of the siRNA molecule comprises nucleotides of about 15 to about 60 in length (e.g., nucleotides of about 15-60, 15-50, 15-40, 15-30, 15-25, or 19-25 in length, or nucleotides of 15, 16, 17, 18, 19, 20, 21, 22, 23, 24, or 25 in length). In one specific embodiment, the siRNA may be chemically synthesized. The siRNA molecule of the present invention can silence expression of a target sequence *in vitro* and/or *in vivo.* In some embodiments, the siRNA may not contain modified nucleotides; in other embodiments, the siRNA comprises at least one modified nucleotide. For example, the siRNA comprises one, two, three, four, five, six, seven, eight, nine, ten, or more modified nucleotides in the double strand region. dsRNA or pre-RNA molecules include any precursor molecule that is processed *in vivo* by an endonuclease to produce an active siRNA. shRNA is a small hairpin RNA or short hairpin RNA comprising short RNA sequences producing tight hairpin turns that can be used to silence gene expression via RNA interference. The shRNA hairpin structure can be lysed by a cellular machinery to siRNA. A miRNA (microRNA) is a single strand RNA molecule regulating gene expression with about 21-23 nucleotides in length.

The term "inhibit expression of a target gene" refers to the ability of the interfering RNA (e.g., siRNA) of the present invention to silence, reduce, or inhibit expression of a target gene (e.g., a B7-H3 gene).

The term "therapeutically effective amount" refers to an amount of a subject compound that will elicit the biological or medical response in a tissue, system, or subject that is being sought by a researcher, a veterinarian, a medical doctor, or other clinicians. The term "therapeutically effective amount" includes the amount of an active ingredient as follows: when administered, it is sufficient to prevent progression of one or more signs or symptoms of a disorder or disease treated, or to some extent, ameliorate one or more signs or symptoms of a disorder or disease treated. The therapeutically effective amount will vary with active ingredients, the disease to be treated and its severity, as well as subject's age, weight, sex, etc.

The term "subject" can be a mammal, e.g., human, monkey, dog, rabbit, mouse, rat, etc.; in one embodiment of the present invention, the subject described above is a human.

The term "comprises" or "comprising" is an open-ended description that includes the specified ingredients or steps as described, as well as other specified ingredients or steps that do not substantially affect the technical effect. When the term is used to describe a sequence of a protein or nucleic acid, the protein or nucleic acid may consist of the sequence, or may have additional amino acids or nucleotides at one or two ends of the protein or nucleic acid, while retaining the same or similar activity as the original sequence. For example, SEQ ID NOs: 1, 2, and 3 of the present application are the target site sequences of siRNA for targeting B7-H3. Since the length of the siRNA can be 15-60 nt, preferably 19-23 nt, several nt of nucleotides at both ends or one end of SEQ ID NOs: 1, 2, and 3 are reduced, or several nt or several tens of nt of nucleotides at both ends or one segment of SEQ ID NOs: 1, 2, and 3 are added, and the siRNA still falls within the protection scope of the present application as long as it has activity to silence B7-H3. Therefore, it is well known to those skilled in the art that the choice of a target sequence lies primarily in the position and not only in the actual length.

### BRIEF DESCRIPTION OF THE DRAWINGS

FIG. 1 shows a mass spectrum of the sense strand (upper figure) and the antisense strand (lower figure) of siRNA with a target sequence consisting of the nucleotide sequence set forth in SEQ ID NO: 1.
FIG. 2 shows a mass spectrum of the sense strand (upper figure) and the antisense strand (lower figure) of siRNA with a target sequence consisting of the nucleotide sequence set forth in SEQ ID NO: 2.
FIG. 3 shows a mass spectrum of the sense strand (upper figure) and the antisense strand (lower figure) of siRNA with a target sequence consisting of the nucleotide sequence set forth in SEQ ID NO: 3.
FIG. 4 shows the inhibition effect of siRNA-1, siRNA-2, and siRNA-3 on B7-H3 gene in 293T cells, of which Blank is a blank control of equal volume of Opti-MEM culture medium, Mock is a control of a transfection reagent without siRNA, and siNC-1 is a negative control purchased from Guangzhou RiboBio Co., Ltd. (Cat. No.: siN0000001-1-5).
FIG. 5 shows the inhibition effect of siRNA-1 to siRNA-25 on B7-H3 gene in A375 cells, of which Blank is a blank control of equal volume of Opti-MEM culture medium, Mock is a control of a transfection reagent without siRNA, and siNC-1 and siNC-2 are negative controls (siNC-1 is purchased from Guangzhou RiboBio Co., Ltd., and siNC-2 is purchased from Beijing Tsingke Biotechnology Co., Ltd.).
FIG. 6 shows a mass spectrum of the sense strand of siRNA-4.
FIG. 7 shows a mass spectrum of the antisense strand of siRNA-4.
FIG. 8 shows a mass spectrum of the sense strand of siRNA-5.
FIG. 9 shows a mass spectrum of the antisense strand of siRNA-5.
FIG. 10 shows a mass spectrum of the sense strand of siRNA-6.
FIG. 11 shows a mass spectrum of the antisense strand of siRNA-6.
FIG. 12 shows a mass spectrum of the sense strand of siRNA-7.
FIG. 13 shows a mass spectrum of the antisense strand of siRNA-7.
FIG. 14 shows a mass spectrum of the sense strand of siRNA-8.
FIG. 15 shows a mass spectrum of the antisense strand of siRNA-8.
FIG. 16 shows a mass spectrum of the sense strand of siRNA-9.
FIG. 17 shows a mass spectrum of the antisense strand of siRNA-9.
FIG. 18 shows a mass spectrum of the sense strand of siRNA-10.
FIG. 19 shows a mass spectrum of the antisense strand of siRNA-10.
FIG. 20 shows a mass spectrum of the sense strand of siRNA-11.
FIG. 21 shows a mass spectrum of the antisense strand of siRNA-11.
FIG. 22 shows a mass spectrum of the sense strand of siRNA-12.
FIG. 23 shows a mass spectrum of the antisense strand of siRNA-12.
FIG. 24 shows a mass spectrum of the sense strand of siRNA-13.
FIG. 25 shows a mass spectrum of the antisense strand of siRNA-13.
FIG. 26 shows the inhibitory effect of different types of siB7-H3 on B7-H3 expression.
FIG. 27 shows the effective particle size of LNP-siB7-H3-m4.
FIG. 28 is a bar graph showing the inhibition of B7-H3 gene expression in PC-3 or RM-1 by LNP-siB7-H3.
FIG. 29 shows the inhibition rate of tumor growth in LNP-siB7-H3-m4 groups with different doses.

### DETAILED DESCRIPTION

Unless otherwise defined, all scientific and technical terms used in the present invention have the same meaning as commonly understood by those skilled in the art to which the present invention relates.

The technical solutions of the present invention will be clearly and completely described below with reference to the examples of the present invention, and it is obvious that the described examples are only a part of the examples of the present invention but not all of them. Based on the examples of the present invention, all other examples obtained by those of ordinary skills in the art without creative work shall fall within the protection scope of the present invention.

In the present application, to test the extent of gene silencing, a test sample (e.g., a biological sample from a target organism expressing a target gene or a sample of cells expressing a target gene in culture) is contacted with an interfering RNA (e.g., siRNA) that silences, reduces, or inhibits expression of a target gene. Expression of the target gene in the test sample is compared to expression of the target gene in a sample that is not contacted with the interfering RNA (e.g., siRNA), and a control sample (e.g., a sample expressing a target gene) can be set to a value of 100%. In specific embodiments, silencing, inhibition, or reduction of expression of the target gene is achieved when the test sample has a value of about 95%, 90%, 85%, 80%, 75%, 70%, 65%, 60%, 55%, 50%, 45%, 40%, 35%, 30%, 25%, 20%, 10%, 5%, or 0% relative to the control sample. Suitable assays include, but are not limited to, assays for protein or mRNA levels using techniques known to those skilled in the art, such as dot blot, Northern blot, real-time RT-PCR, *in situ* hybridization, ELISA, immunoprecipitation, enzyme function, and phenotypic assays known to those skilled in the art.

B7-H3 siRNA (Nos. 1-3) was synthesized by Guangzhou RiboBio Co., Ltd., and B7-H3 siRNA (Nos. 4-25) was synthesized by Beijing Tsingke Biotechnology Co., Ltd., with the target sequences and positions shown in Table 1 and the siRNA sequences shown in Table 2.

**Table 1. B7-H3 target sequences**

| No. | Position | Target sequence | SEQ ID NO: |
|---|---|---|---|
| 1 | 720 | CAACGAGCAGGGCTTGTTT | SEQ ID NO: 1 |
| 2 | 1551 | GTCTGTCTGTCTCATTGCA | SEQ ID NO: 2 |
| 3 | 1595 | GCTGGAGAAAGATCAAACA | SEQ ID NO: 3 |
| 4 | 987 | GCAGCTGACAGACACCAAA | SEQ ID NO: 4 |
| 5 | 1011 | GGTGCACAGTTTCACCGAA | SEQ ID NO: 5 |
| 6 | 1875 | AGACCACTGTGCAGCCTTA | SEQ ID NO: 6 |
| 7 | 2645 | AGATGTCAGCACTGTGTTA | SEQ ID NO: 7 |
| 8 | 1585 | GCTTTCGTGTGCTGGAGAA | SEQ ID NO: 8 |
| 9 | 1710 | AGAAGATGATGGACAAGAA | SEQ ID NO: 9 |
| 10 | 1713 | AGATGATGGACAAGAAATA | SEQ ID NO: 10 |
| 11 | 1901 | CAATGGACATGATTCCCAA | SEQ ID NO: 11 |
| 12 | 1905 | GGACATGATTCCCAAGTCA | SEQ ID NO: 12 |
| 13 | 1971 | CCTTAGTTCTCTAAGTCAT | SEQ ID NO: 13 |
| 14 | 2109 | GATGCAATATTCAGACTGA | SEQ ID NO: 14 |
| 15 | 2314 | CCTTCCTCCTCTTGCTCTA | SEQ ID NO: 15 |
| 16 | 2615 | CCATTCAGTTGATGTTTAT | SEQ ID NO: 16 |
| 17 | 2622 | GTTGATGTTTATTGAGCAA | SEQ ID NO: 17 |
| 18 | 2625 | GATGTTTATTGAGCAACTA | SEQ ID NO: 18 |
| 19 | 2688 | GATAAAGTTCCTCCCTCAA | SEQ ID NO: 19 |
| 20 | 2721 | GCTGGGAGACAGACAACTA | SEQ ID NO: 20 |
| 21 | 2725 | GGAGACAGACAACTAACTA | SEQ ID NO: 21 |
| 22 | 2727 | AGACAGACAACTAACTACA | SEQ ID NO: 22 |
| 23 | 2806 | GCTCAAGGCTTCCTGGATA | SEQ ID NO: 23 |
| 24 | 3139 | GGTAGAGTGAGACTTCAGA | SEQ ID NO: 24 |
| 25 | 3209 | GTGAGGACTTCTAATTTAA | SEQ ID NO: 25 |

**Table 2. B7-H3 siRNA sequences**

| No. | Position | SEQ ID NO: | Sense strand | SEQ ID NO: | Antisense strand |
|---|---|---|---|---|---|
| 1 | 720 | SEQ ID NO: 26 | CAACGAGCAGGGCUU GUUUdTdT | SEQ ID NO: 27 | AAACAAGCCCUGCUCG UUGdTdT |
| 2 | 1551 | SEQ ID NO: 28 | GUCUGUCUGUCUCAU UGCAdTdT | SEQ ID NO: 29 | UGCAAUGAGACAGACA GACdTdT |
| 3 | 1595 | SEQ ID NO: 30 | GCUGGAGAAAGAUCA AACAdTdT | SEQ ID NO: 31 | UGUUUGAUCUUUCUCC AGCdTdT |
| 4 | 987 | SEQ ID NO: 32 | GCAGCUGACAGACACC AAAdTdT | SEQ ID NO: 33 | UUUGGUGUCUGUCAGC UGCdTdT |
| 5 | 1011 | SEQ ID NO: 34 | GGUGCACAGUUUCAC CGAAdTdT | SEQ ID NO: 35 | UUCGGUGAAACUGUGC ACCdTdT |
| 6 | 1875 | SEQ ID NO: 36 | AGACCACUGUGCAGCC UUAdTdT | SEQ ID NO: 37 | UAAGGCUGCACAGUGG UCUdTdT |
| 7 | 2645 | SEQ ID NO: 38 | AGAUGUCAGCACUGU GUUAdTdT | SEQ ID NO: 39 | UAACACAGUGCUGACA UCUdTdT |
| 8 | 1585 | SEQ ID NO: 40 | GCUUUCGUGUGCUGG AGAAdTdT | SEQ ID NO: 41 | UUCUCCAGCACACGAA AGCdTdT |
| 9 | 1710 | SEQ ID NO: 42 | AGAAGAUGAUGGACA AGAAdTdT | SEQ ID NO: 43 | UUCUUGUCCAUCAUCU UCUdTdT |
| 10 | 1713 | SEQ ID NO: 44 | AGAUGAUGGACAAGA AAUAdTdT | SEQ ID NO: 45 | UAUUUCUUGUCCAUCA UCUdTdT |
| 11 | 1901 | SEQ ID NO: 46 | CAAUGGACAUGAUUC CCAAdTdT | SEQ ID NO: 47 | UUGGGAAUCAUGUCCA UUGdTdT |
| 12 | 1905 | SEQ ID NO: 48 | GGACAUGAUUCCCAA GUCAdTdT | SEQ ID NO: 49 | UGACUUGGGAAUCAUG UCCdTdT |
| 13 | 1971 | SEQ ID NO: 50 | CCUUAGUUCUCUAAG UCAUdTdT | SEQ ID NO: 51 | AUGACUUAGAGAACUA AGGdTdT |
| 14 | 2109 | SEQ ID NO: 52 | GAUGCAAUAUUCAGA CUGAdTdT | SEQ ID NO: 53 | UCAGUCUGAAUAUUGC AUCdTdT |
| 15 | 2314 | SEQ ID NO: 54 | CCUUCCUCCUCUUGCU CUAdTdT | SEQ ID NO: 55 | UAGAGCAAGAGGAGG AAGGdTdT |
| 16 | 2615 | SEQ ID NO: 56 | CCAUUCAGUUGAUGU UUAUdTdT | SEQ ID NO: 57 | AUAAACAUCAACUGAA UGGdTdT |
| 17 | 2622 | SEQ ID NO: 58 | GUUGAUGUUUAUUGA GCAAdTdT | SEQ ID NO: 59 | UUGCUCAAUAAACAUC AACdTdT |
| 18 | 2625 | SEQ ID NO: 60 | GAUGUUUAUUGAGCA ACUAdTdT | SEQ ID NO: 61 | UAGUUGCUCAAUAAAC AUCdTdT |
| 19 | 2688 | SEQ ID NO: 62 | GAUAAAGUUCCUCCC UCAAdTdT | SEQ ID NO: 63 | UUGAGGGAGGAACUU UAUCdTdT |
| 20 | 2721 | SEQ ID NO: 64 | GCUGGGAGACAGACA ACUAdTdT | SEQ ID NO: 65 | UAGUUGUCUGUCUCCC AGCdTdT |
| 21 | 2725 | SEQ ID NO: 66 | GGAGACAGACAACUA ACUAdTdT | SEQ ID NO: 67 | UAGUUAGUUGUCUGUC UCCdTdT |
| 22 | 2727 | SEQ ID NO: 68 | AGACAGACAACUAAC UACAdTdT | SEQ ID NO: 69 | UGUAGUUAGUUGUCU GUCUdTdT |
| 23 | 2806 | SEQ ID NO: 70 | GCUCAAGGCUUCCUG GAUAdTdT | SEQ ID NO: 71 | UAUCCAGGAAGCCUUG AGCdTdT |
| 24 | 3139 | SEQ ID NO: 72 | GGUAGAGUGAGACUU CAGAdTdT | SEQ ID NO: 73 | UCUGAAGUCUCACUCU ACCdTdT |
| 25 | 3209 | SEQ ID NO: 74 | GUGAGGACUUCUAAU UUAAdTdT | SEQ ID NO: 75 | UUAAAUUAGAAGUCCU CACdTdT |

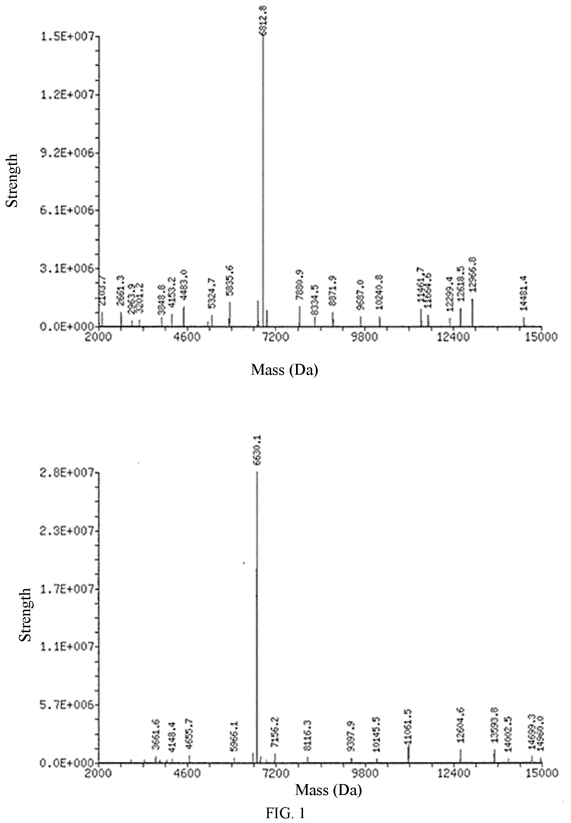

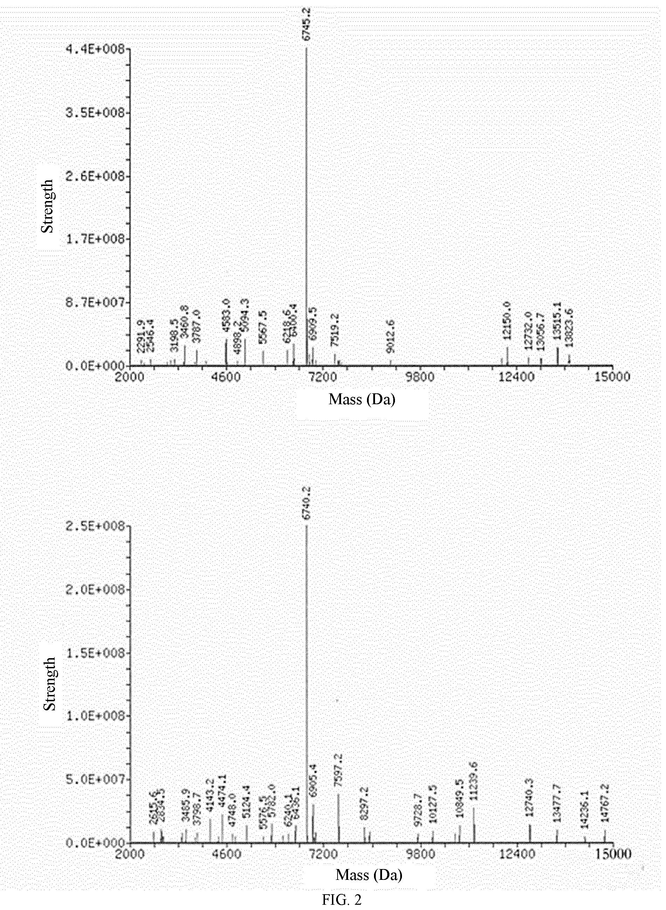

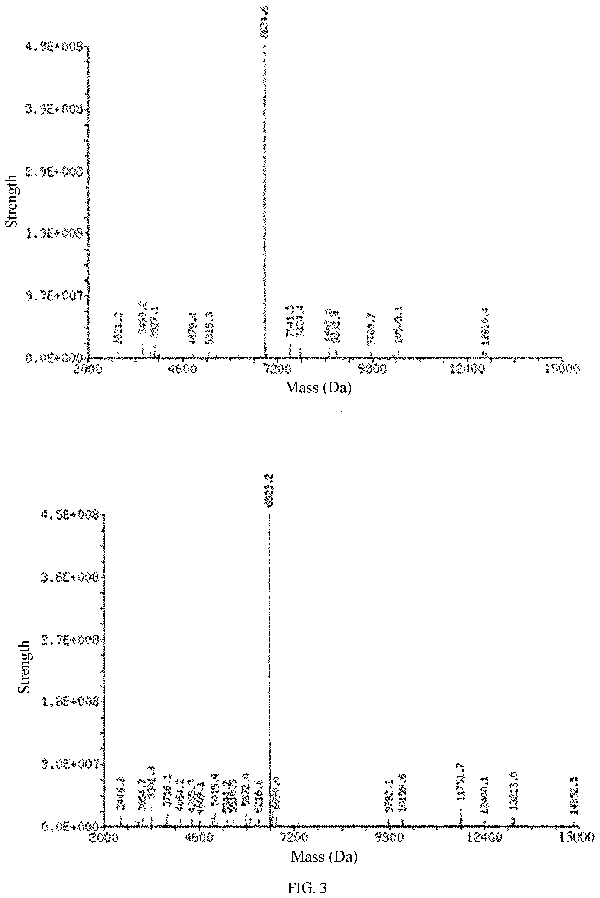

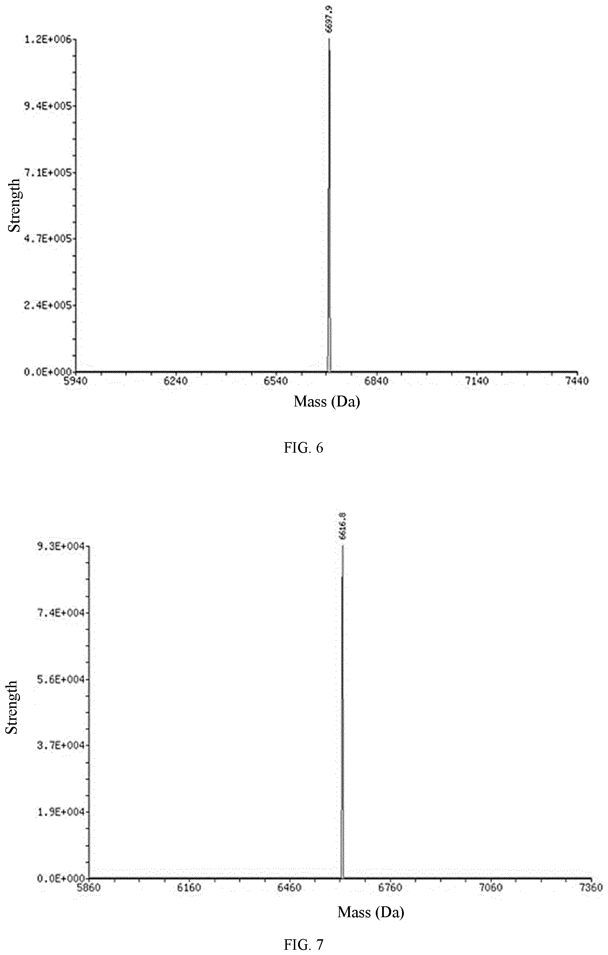

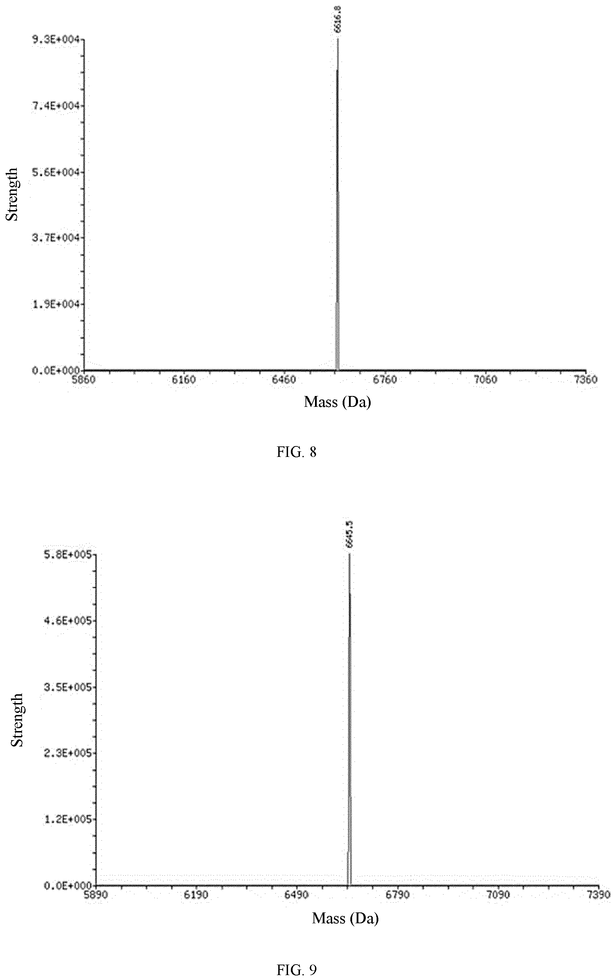

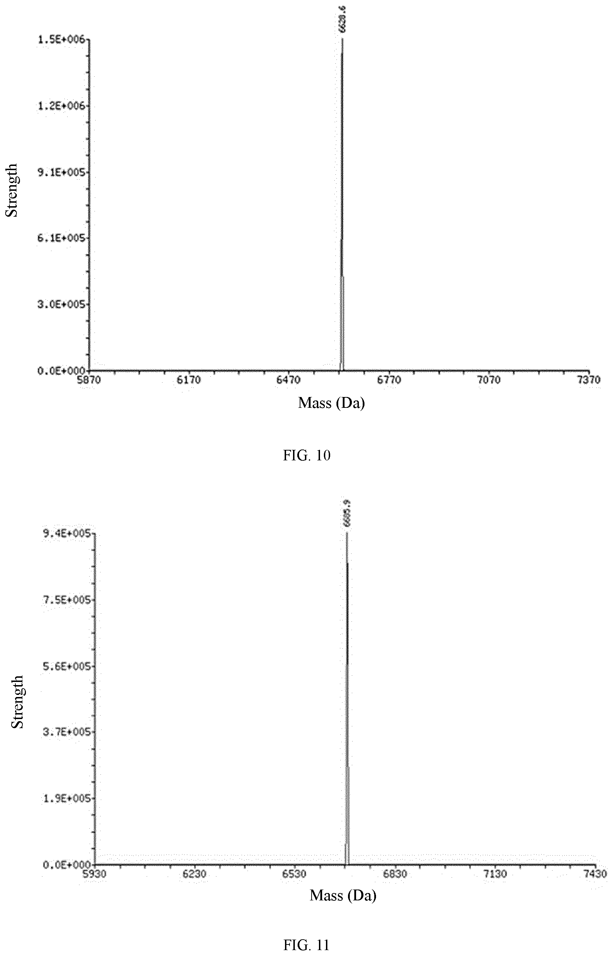

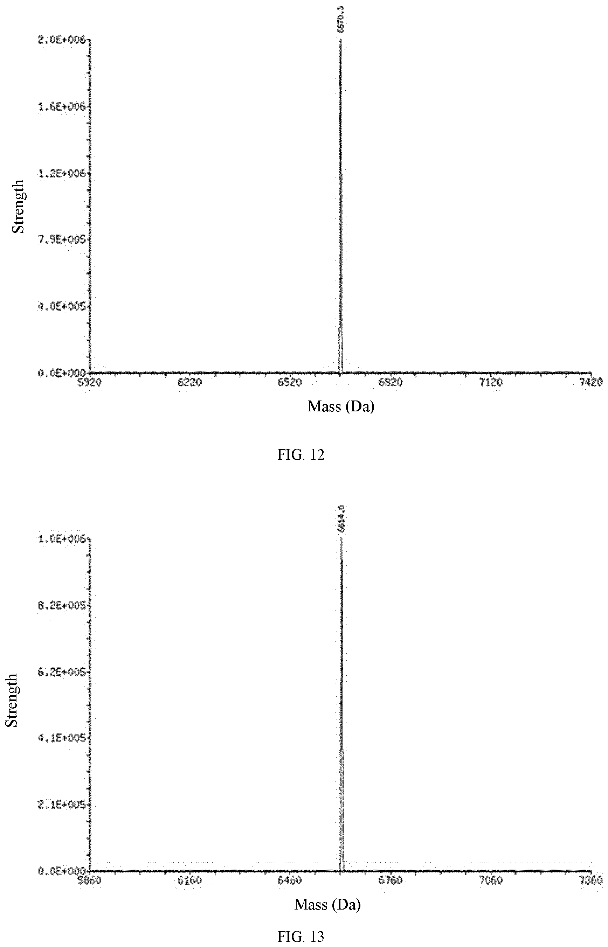

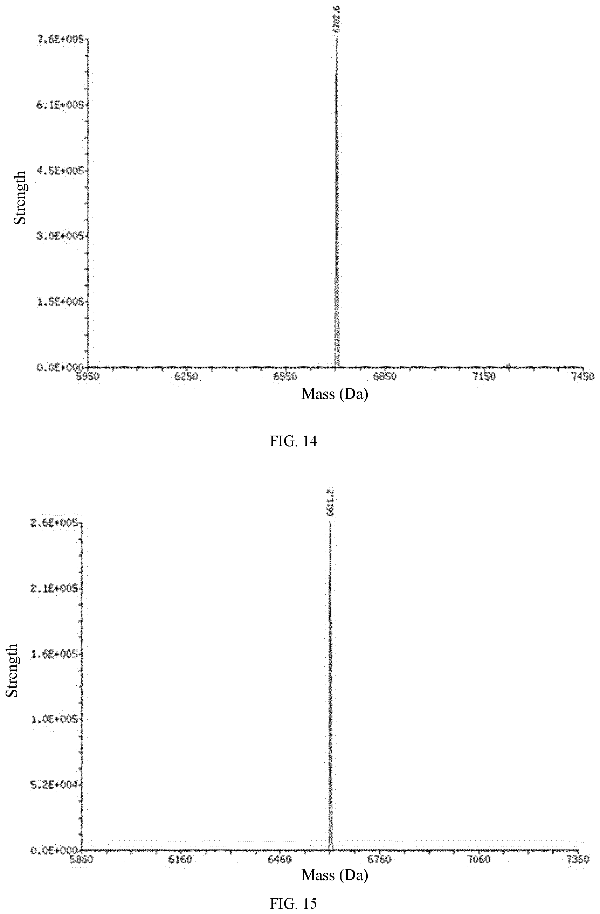

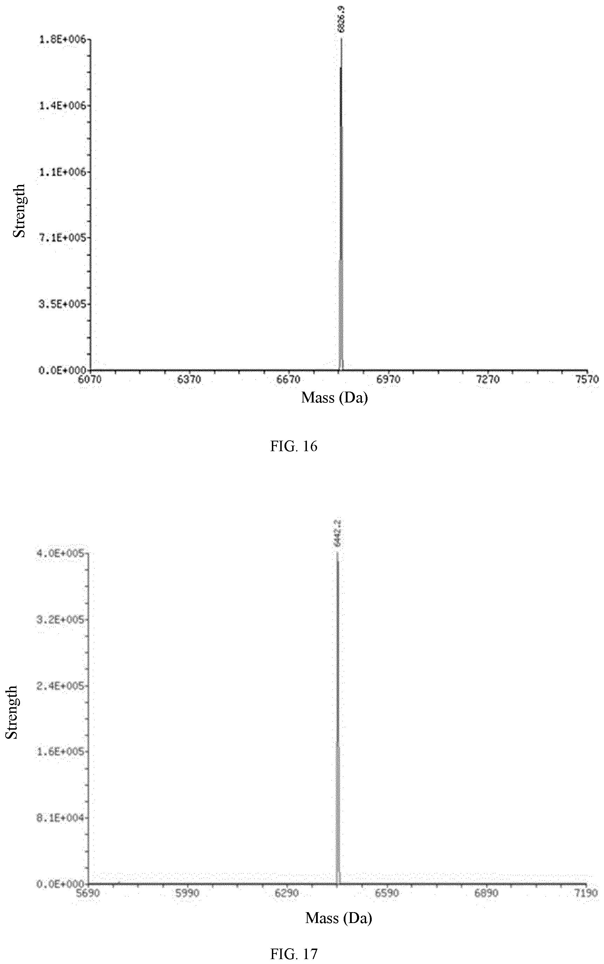

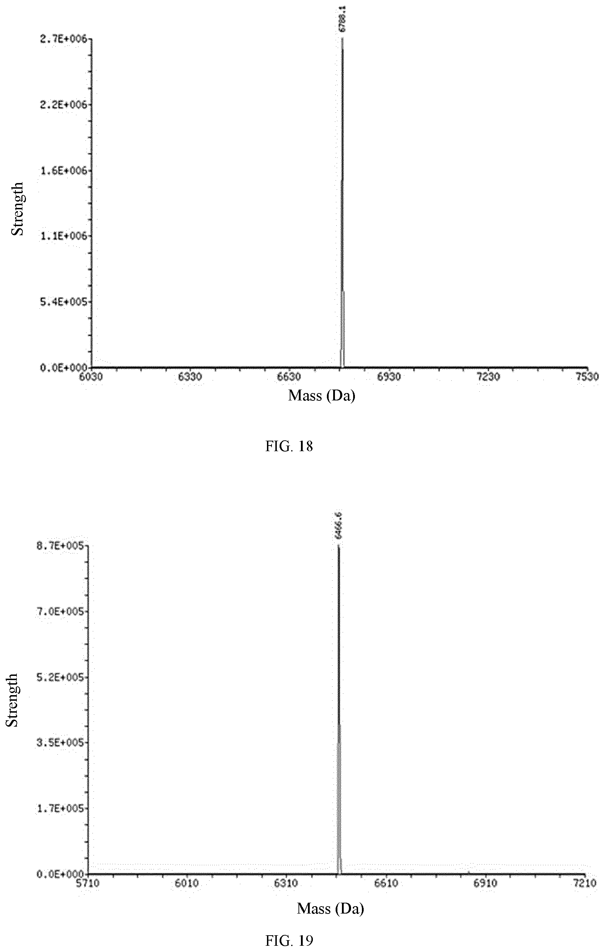

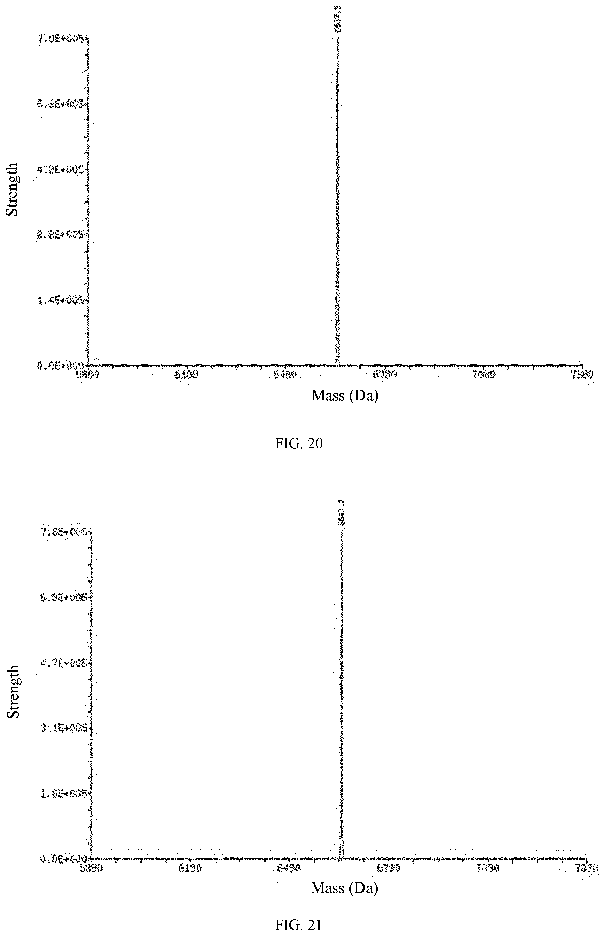

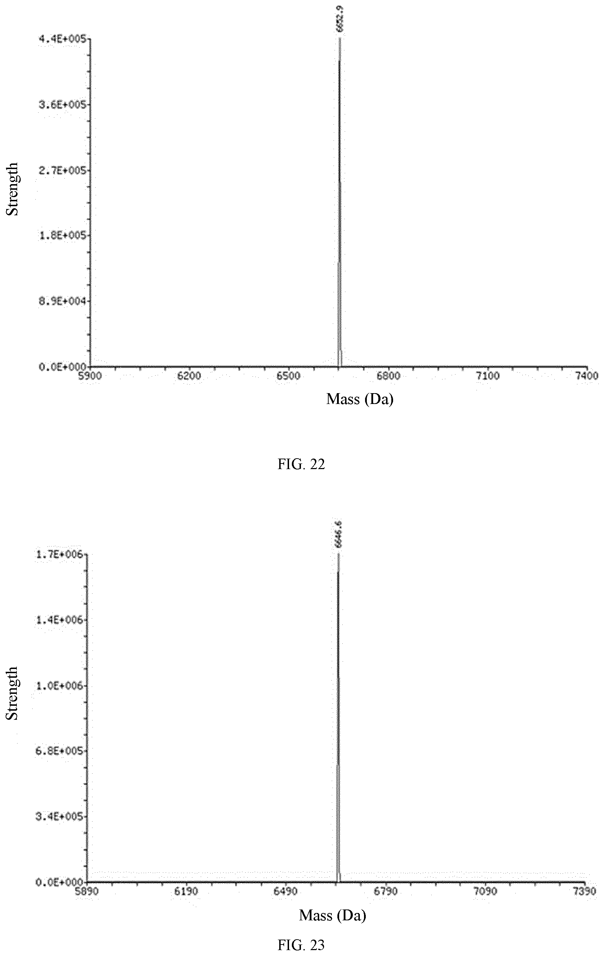

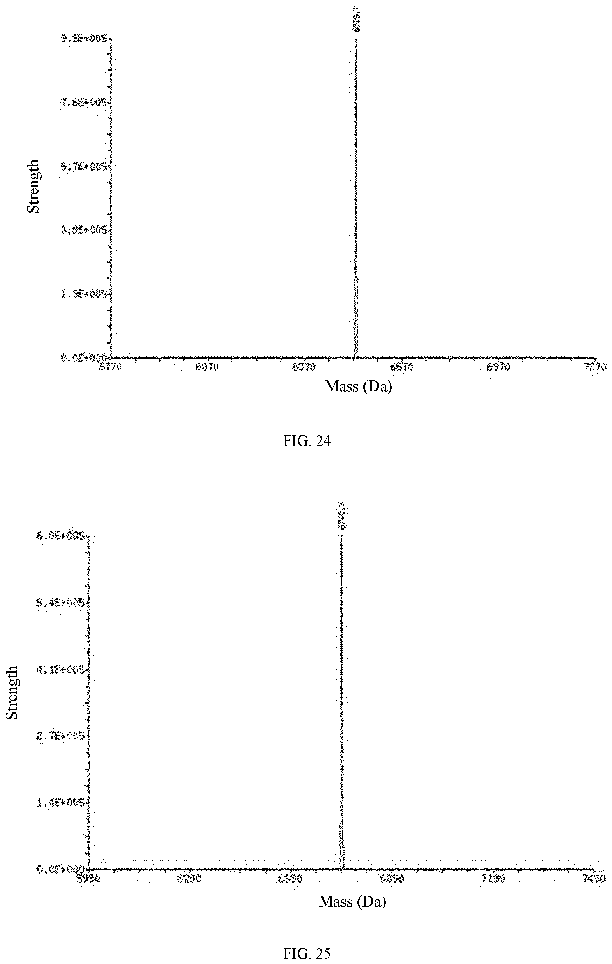

The mass spectrum of the sense strand and the antisense strand of siRNA with a target sequence consisting of the nucleotide sequence set forth in SEQ ID NO: 1, SEQ ID NO: 2, or SEQ ID NO: 3 is shown in FIGs 1-3, and the molecular weight of the siRNA is 13441.41, 13482.5, and 13355.28, respectively. FIGs. 6-25 show the mass spectra of the sense strands and the antisense strands of siRNA-4 to siRNA-13, respectively.

### Example 1: Inhibitory Effect of siRNA in 293T Cells

1. The siRNA targeting the sequence shown in SEQ ID NO: 1, SEQ ID NO: 2, or SEQ ID NO: 3 were transfecte separately into 293T cells, and the sense strands and the antisense strands of the siRNA are shown in Table 2.
1) On day 1, 293T cells were seeded into a 12-well plate at 2 × 10⁵ cells/well, and cultured in a 1 mL antibiotic-free culture medium containing 10% FBS (fetal bovine serum) at 37 °C, 5% CO₂ overnight to allow the cells to adhere to the wall.
2) The siRNA was diluted with 50 µL of Opti-MEM culture medium to a concentration of 100 nM (the negative control group was siNC-1 (purchased from Guangzhou RiboBio Co., Ltd., Cat. No.: siN0000001-1-5) at the same concentration). 3 µL of Lipofectamine^{™} 3000 transfection reagent was diluted with another 50 µL of Opti-MEM culture medium. The two solutions were well mixed, and left to stand at room temperature for 15 min.
3) 900 µL of the culture medium was first added to each well, and subsequently 100 µL of the mixed solution described above was added to obtain a final 1 mL transfection system.
4) After 48 h of culture, the cells were collected for RNA extraction for subsequent detection.

2. RNA extraction
1) Trizol lysis: The sample added with the Trizol solution was incubated at room temperature for 5 min.
2) 0.2-fold volume (0.2 mL/1 mL Trizol) of chloroform was added, and the mixture was vortexed for 15 s and left to stand at room temperature for 5 min.
3) The mixture was centrifuged at 12000 rpm and 4 °C for 15 min, and after liquid separation, the upper aqueous phase was carefully pipetted into a new 1.5 mL centrifuge tube.
4) About 0.6 mL (the same volume as the supernatant) of isopropanol was added, and the mixture was well mixed by allowing the tube to be repeatedly upside down, and then was left to precipitate at -20 °C for more than 1 h.
5) The mixture was centrifuged at 12000 rpm and 4 °C for 30 min, a white precipitate was found at the bottom of the tube, and the supernatant was removed.
6) 1 mL of 75% ethanol was added, and the tube wall was flicked to allow the precipitate to float. The mixture was centrifuged at 12000 rpm and 4 °C for 5 min, and the procedure was repeated once.
7) Centrifugation was carried out for a short time after removing the supernatant, and the residual liquid was pipetted thoroughly. The cover of the centrifuge tube was opened for drying, and then an appropriate amount of RNase-free H₂O was added to dissolve the precipitate after it was dried to a translucent state.

3. Q-PCR detecting
1) The mass and concentration of the RNA were detected using an ultraviolet spectrophotometer, and 1 µg of RNA was reversely transcribed according to the system shown in Table 3:

**Table 3. Reaction system**

| Reagent | **Amount** |
|---|---|
| RTase Mix | 2 µL |
| RT primer^{©} (10 µM) | 1 µL |
| 5×RTase Buffer | 2 µL |
| RNA template | 1 µg |
| Total | 10 µL |

Type of RT primer: (A) Oligo-dT, (B) Random primer, and (C) Gene/miRNA specific primer. The system described above was well mixed and centrifuged to allow the liquid to be all at the bottom of the tube. A reaction program was set as follows: at 42 °C for 60 min and at 72 °C for 10 min. The product was a cDNA template, which was 5-fold diluted for subsequent test.
2) A Q-PCR system was prepared according to Table 4 to detect the level of B7-H3 gene:

**Table 4. Reaction system**

| Reagent | Amount |
|---|---|
| RNase-Free H₂O | 2.6 µL |
| Forword Primer (10µM) | 0.2 µL |
| Reverse Primer (10µM) | 0.2 µL |
| SYBR Green Mix | 5 µL |
| cDNAtemplate | 2 µL |
| Total | 10 µL |

GAPDH was used as an internal reference to detect the gene expression level, and the sequences of the primers used are as follows:
GAPDH-F: TCTGACTTCAACAGCGACAC (SEQ ID NO: 76)
GAPDH-R: GCCAAATTCGTTGTCATACC (SEQ ID NO: 77)
B7-H3-F: GTGTGCTGGAGAAAGATCAAAC (SEQ ID NO: 78)
B7-H3-R: TGGTCAGGCTATTTCTTGTCC (SEQ ID NO: 79)

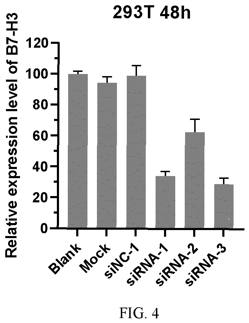

The results in FIG. 4 show that compared to Mock and siNC-1, all three siRNAs targeting B7-H3 can inhibit B7-H3 expression in 293T cells cultured for 48 h after transfection with siRNA. The inhibitory effect of siRNA-1 (inhibition rate 65.7%) and siRNA-3 (inhibition rate 71.0%) was better than that of siRNA-2 (inhibition rate 37.3%).

### Example 2: Inhibitory Effect of siRNA in A375 Cells

1. The siRNAs targeting the sequences shown in SEQ ID NOs: 1-25 were transfected separatedly into A375 cells. The sense strands and the antisense strands of the siRNA are listed in Table 2.
1) On day 1, A375 cells were seeded into a 24-well plate at 8 × 10⁴ cells/well, and cultured in a 500 µL DMEM culture medium containing 10% FBS and 1% Penicillin-Streptomycin at 37 °C, 5% CO₂ for 24 h to allow the cells to adhere to the wall. Before transfection on day 2, the culture medium was changed to a 450 µL antibiotic-free culture medium.
2) The siRNA was diluted with 100 µL of Opti-MEM culture medium, and the negative control groups were siNC-1 purchased from Guangzhou RiboBio Co., Ltd. and siNC-2 purchased from Beijing Tsingke Biotechnology Co., Ltd. at the same concentration. A Lipofectamine^{™}RNAiMAX transfection reagent was diluted with another 100 µL of Opti-MEM culture medium. The two solutions were well mixed, and left to stand at room temperature for 5 min.
3) 50 µL of the mixed solution described above was added to each well to obtain a final 500 µL transfection system, and the final concentration of siRNA was 50 nM.
4) After 48 h of culture, the cells were collected for RNA extraction for subsequent detection.

2. RNA extraction
1) 300 µL of RNA lysate and 300 µL of RNA diluent in an Eastep^{™} Super total RNA extraction kit was added and well mixed, and subsequently transferred to a new 1.5 mL centrifuge tube.
2) The mixture was centrifuged at 14000 g for 5 min, the supernatant was carefully pipetted into a new 1.5 mL centrifuge tube.
3) 0.5-fold volume of absolute ethanol was added, and the mixture was well mixed by allowing the tube to be repeatedly upside down 10-20 times. Subsequently, the mixture was transferred to a centrifuge column and centrifuged at 12000-14000 g for 1 min. The filtrate was discarded.
4) 600 µL of RNA washing liquid was added, and the mixture was centrifuged at 12000-14000 g for 1 min. The filtrate was discarded.
5) 50 µL of DNA enzyme I incubation liquid was added and the mixture was incubated for 15 min.
6) 600 µL of RNA washing liquid was added, the mixture was centrifuged at 12000-14000 g for 1 min, and the filtrate was discarded. The procedure was repeated once. The centrifuge column was placed back in the collection tube, centrifuged at 12000-14000 g for 2 min, and then placed in the collection tube.
7) An appropriate amount of RNase-free H₂O was added, and the mixture was left to stand for 10 min and centrifuged at 12000-14000 g for 1 min. The RNA was collected and preserved at -70 °C.

3. Q-PCR detecting procedure
1) Used a NanoDrop^{™} to measure the the quality and concentration of RNA. Took 1 µg of RNA and added RNase-free H₂O to a volume of 10 µL. Incubated at 70 °C for 5 min then cooled on ice for 5 min.
2) A reaction system was established according to Table 5:

**Table 5. Reaction system**

| Reagent | Amount |
|---|---|
| GoScript^{™}ReactionBuffer,Oliga (dT) | 4 µL |
| GoScript^{™} Enzyme Mix | 2 µL |
| RNA template | 10 µL |
| RNase-free H₂O | Supplement to 20 µL |
| Total | 20 µL |

3) Mixed the reaction system thoroughly and centrifuged to collect the liquid at the bottom of the tube. Incubated at 25 °C for 5 min, 42 °C for 60 min, and 72 °C for 15 min. The product was the cDNA.
4) A Q-PCR system was prepared according to Table 6 to detect the level of B7-H3 gene using the same primers as in Example 1:

**Table 6. Reaction system**

| Reagent | Amount |
|---|---|
| 2×TB Green^{®} Premix Ex Taq^{™} II | 10 µL |
| Forward primer (10µM) | 0.5 µL |
| Reverse primer (10µM) | 0.5 µL |
| cDNA | 1.2 µL |
| RNase-free H₂O | 7.8 µL |
| Total | 20 µL |

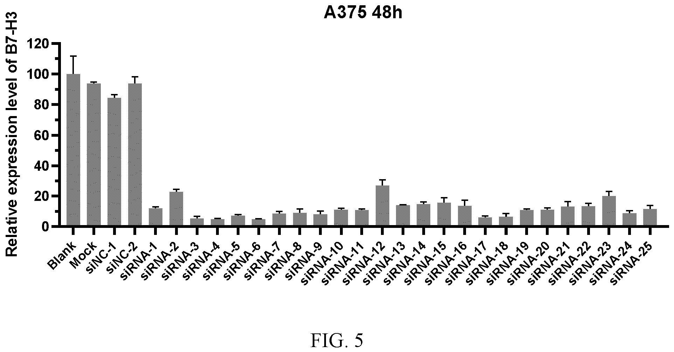

The results in FIG. 5 show that compared to Mock and siNC, all siRNAs targeting B7-H3 can inhibit B7-H3 expression in A375 cells after transfection with siRNA. siRNA-3 (inhibition rate 94.6%), siRNA-4 (inhibition rate 95.0%), siRNA-5 (inhibition rate 92.7%), siRNA-6 (inhibition rate 94.9%), siRNA-17 (inhibition rate 94.0%), and siRNA-18 (inhibition rate 93.4%) showed good inhibitory effect.

### Example 3: siB7-H3 Modified Sequence Inhibiting Gene Expression

1. The siRNA-4 sequence was chemically modified according to the methods in the following table:

| **No.** | SEQ ID NO: | **Sense (5'-3')** | SEQ ID NO: | **Antisense (5'-3')** | **Modification method** |
|---|---|---|---|---|---|
| siB7-H3-m1 | SEQ ID NO: 80 | 5'- G(mC)AG(mC)(mU)GA(mC)A GA(mC)A(mC)(mC)AAAdTd T-3' | SEQ ID NO: 81 | 5'- (mU)(mU)(mU)GG(mU) G(mU)(mC)(mU)G(mU)( mC)AG(mC)(mU)G(mC) dTdT-3' | Methylation |
| siB7-H3-m2 | SEQ ID NO: 82 | 5'- (mG)(mC)(mA)(mG)(mC)(m U)(mG)(mA)(mC)(fA)(fG)(f | SEQ ID NO: 83 | 5'- (mU)(mU)(mU)(mG)(m G)(mU)(mG)(mU)(mC)( | Methylation and fluorination |
| | | A)(mC)(mA)(mC)(mC)(mA)( mA)(mA) dTdT-3' | | mU)(mG)(mU)(mC)(mA )(mG)(mC)(mU)(mG)(m C) dTdT-3' | |
| siB7-H3-m3 | SEQ ID NO: 84 | 5’- (mG)-(mC)- (mA)(mG)(mC)(mU)(mG)(m A)(mC)(fA)(fG)(fA)(mC)(m A)(mC)(mC)(mA)(mA)(mA) dTdT-3’ | SEQ ID NO: 85 | 5'- (mU)-(mU)- (mU)(mG)(mG)(mU)(m G)(mU)(mC)(mU)(mG)( mU)(mC)(mA)(mG)(mC) (mU)-(mG)-(mC) dTdT- 3' | Thiophosphor ylation, methylation, and fluorination |
| siB7-H3-m4 | SEQ ID NO: 86 | 5'- GCAGC(Ψ)GACAGACACC AAA dTdT-3' | SEQ ID NO: 87 | 5’- (Ψ)(Ψ)(Ψ)GG(Ψ)G(Ψ)C( Ψ)G(Ψ)CAGC(Ψ)GC dTdT-3’ | Pseudouridine |
| siB7-H3-m5 | SEQ ID NO: 88 | 5'- (mG)(mC)(mA)(fG)(mC)(fU) (mG)(fA)(mC)(fA)(fG)(fA)( mC)(fA)(mC)(fC)(mA)(mA)( mA) dTdT-3' | SEQ ID NO: 89 | 5'- (fU)(fU)(fU)(mG)(fG)(m U)(fG)(mU)(fC)(mU)(m G)(mU)(fC)(mA)(fG)(m C)(fU)(fG)(fC) dTdT-3' | Fluorination and methylation |
| siB7-H3-m6 | SEQ ID NO: 90 | 5'- (mG)-(mC)- (mA)(fG)(mC)(fU)(mG)(fA)( mC)(fA)(fG)(fA)(mC)(fA)(m C)(fC)(mA)(mA)(mA) dTdT- 3' | SEQ ID NO: 91 | 5’- (fU)-(fU)- (fU)(mG)(fG)(mU)(fG)( mU)(fC)(mU)(mG)(mU)( fC)(mA)(fG)(mC)(fU)- (fG)-(fC) dTdT-3’ | Fluorination, methylation, and thiophosphory lation |

| | | | | | |
|---|---|---|---|---|---|
| m stands for a methyl modification (2'-O-methyl); f stands for a fluorination modification (2'-deoxy-2'-fluoro); - stands for a thiophosphorylation modification (phosphorothioate); Ψ stands for a pseudouridine modification (pseudouridine). | | | | | |

2. PC-3 cells were transfected with the modified sequences
1) On day 1, PC-3 cells were seeded into a 24-well plate at 1.5 × 10⁵ cells/well, and cultured in a 500 µL F12K culture medium containing 10% FBS and 1% Penicillin-Streptomycin at 37 °C, 5% CO₂ for 24 h to allow the cells to adhere to the wall. Before transfection on day 2, the culture medium was changed to a 450 µL antibiotic-free culture medium.
2) The siRNA was diluted with 100 µL of Opti-MEM culture medium, and the positive control was siRNA-4 at the same concentration, and the negative control was siNC at the same concentration. A Lipofectamine^{™}RNAiMAX transfection reagent was diluted with another 100 µL of Opti-MEM culture medium. The two solutions were well mixed, and left to stand at room temperature for 5 min.
3) 50 µL of the mixed solution described above was added to each well to obtain a final 500 µL transfection system, and the final concentration of siRNA was 50 nM.
4) After 48 h of culture, the cells were collected for RNA extraction for subsequent detection.

3. Detection of the expression level of B7-H3

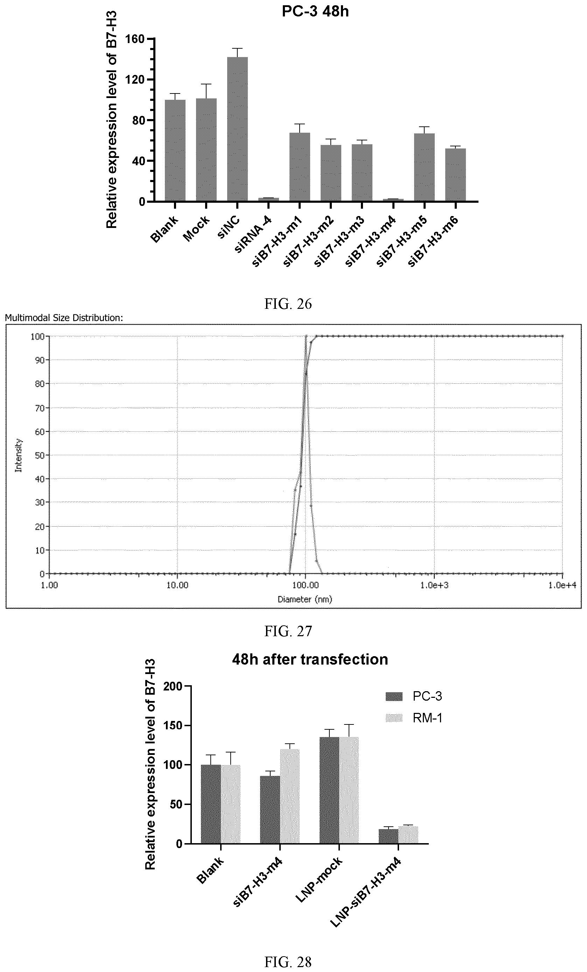

According to the method in Example 2, the total RNA of the cells was extracted and the expression level of B7-H3 was detected by qPCR. The results in FIG. 26 show that compared to Mock and siNC, all six kinds of modified siB7-H3 can inhibit B7-H3 expression in PC-3 cells cultured for 48 h after transfection. siB7-H3-m4 showed the best inhibitory effect (inhibition rate 97.3%), followed by siB7-H3-m6 (inhibition rate 47.9%), siB7-H3-m2 (inhibition rate 44.5%), siB7-H3-m3 (inhibition rate 43.6%), siB7-H3-m5 (inhibition rate 33.1%), and siB7-H3-m1 (inhibition rate 32.5%).

### Example 4: Preparation of LNP-siB7-H3

1) Preparation of each component:
   213.06 mg of SM-102 (Jenkem) was weighed and dissolved by adding 10 mL of anhydrous EtOH to obtain an SM-102 solution; 748.5 mg of M-DMG-2000 (Jenkem) was weighed and dissolved by adding 10 mL of anhydrous EtOH to obtain a PEG solution; 237.06 mg of DSPC (Sigma) was weighed and dissolved by adding 10 mL of anhydrous EtOH to obtain a DSPC solution; 115.98 mg of cholesterol (Sigma) was weighed and dissolved by adding 10 mL of anhydrous EtOH to obtain a cholesterol solution.
2) 2160 µL of SM-102 solution, 64.8 µL of PEG solution, 432 µL of DSPC solution, and 1664 µL of cholesterol solution were well mixed to obtain an LNP/EtOH solution. 450 nmol of siB7-H3-m4 was added with 6.5 mL of 50 mM citric acid buffer (pH = 4.0) to obtain a siB7-H3-m4/buffer solution.
3) Preparation of microfluidic system: The LNP/EtOH solution was treated in tube 1 with a solution volume of 2 mL and a flow rate of 5 mL/min, and the siB7-H3-m4/buffer solution was treated in tube 2 with a solution volume of 6 mL and a flow rate of 15 mL/min. 4 mL of the microfluidic preparation solution was taken and dialyzed with PBS (pH = 7.4) for 16 h to obtain LNP-siB7-H3-m4, and the final concentration of siB7H3 was 0.45 mg/mL. Upon detection by a particle size analyzer NanoBrook 90Plus PALS, LNP-siB7-H3-m4 had an effective particle size of 98.26 nm and a polydispersity index of 0.033 (FIG. 27).

### Example 5: LNP-siB7-H3 Inhibiting Gene Expression

1) On day 1, PC-3 cells were seeded into a 24-well plate at 1.5 × 10⁵ cells/well, and cultured in a 500 µL F12K culture medium containing 10% FBS and 1% Penicillin-Streptomycin. RM-1 cells were seeded into a 24-well plate at 5 × 10⁴ cells/well, and cultured in a 500 µL RPMI 1640 culture medium containing 10% FBS and 1% Penicillin-Streptomycin. The cell culture plate was placed in an incubator with 5% CO₂ at 37 °C for 24 h to allow the cells to adhere to the wall. Three replicate wells were made for each group.
2) On day 2, the culture medium was changed to a 450 µL antibiotic-free culture medium.
3) LNP-siB7-H3-m4 was diluted with an appropriate amount of Opti-MEM culture medium, and the control group was LNP without siRNA and siB7-H3-m4 without delivery system at the same concentration.
4) 50 µL of the solution described above was added to each well to obtain a final 500 µL transfection system, and the working concentration of siRNA was 50 nM.
5) After 48 h of culture after transfection, the cells were collected.
6) According to the method in Example 2, the total RNA of the cells was extracted and the level of B7-H3 mRNA was detected.

The results in FIG. 28 show that LNP-siB7-H3-m4 prepared in Example 4 can inhibit B7-H3 expression in PC-3 cells and RM-1 cells.

### Example 6: LNP-siB7H3 Inhibiting Mouse Tumor Growth

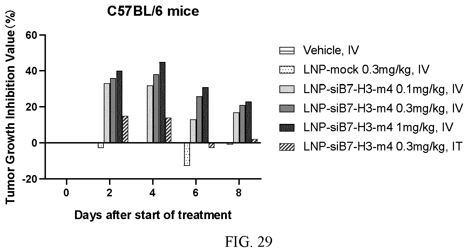

6-8 weeks old male C57BL/6 mice were kept in individual ventilation cages at constant temperature (20-26 °C) and humidity (40-70%). Animal have free access to irradiation-sterilized food during the entire study period. Each mouse was inoculated subcutaneously on the right flank with RM-1 tumor cells in an exponential growth phase. When the tumors volume reached approximately 100 mm³, the mice were randomized into 6 groups and each test agent treatment group have 6 tumor-bearing mice: Vehicle group, intravenous injection of LNP-mock, intravenous injection of low-dose LNP-siB7-H3-m4 (0.1 mg/kg, IV), medium-dose LNP-siB7-H3-m4 medium (0.3 mg/kg, IV), high-dose LNP-siB7-H3-m4 (1 mg/kg, IV), and intratumoral injection of medium dose LNP-siB7-H3 (0.3 mg/kg, IT). Tumor volume was measured after a single administration. The results demonstrated that LNP-siB7-H3-m4 exerted an inhibitory effect on tumor growth, with this effect showing dose-dependency. By the fourth day after administration, the high dose group achieved a tumor inhibition rate of 40% (FIG. 29).

The above description is only for the purpose of illustrating the preferred examples of the present invention, and is not intended to limit the scope of the present invention. Any modifications, equivalents, and the like made without departing from the spirit and principle of the present invention shall fall in the protection scope of the present invention.

The foregoing examples and methods described herein can vary based on the abilities, experience, and preferences of those skilled in the art.

The certain order in which the steps of the method are listed in the present invention does not constitute any limitation to the order of the steps of the method.

## Claims

1. An interfering RNA targeting B7-H3, wherein a target sequence of the interfering RNA comprises a nucleotide sequence set forth in any one or two or more of SEQ ID NOs: 1-25.

2. The interfering RNA according to claim 1, wherein the interfering RNA is selected from siRNA, dsRNA, shRNA, aiRNA, miRNA, and combinations thereof.

3. The interfering RNA according to claim 1 or 2, further comprising an over-hang, preferably, the interfering RNA comprising 1-10 over-hangs.

4. The interfering RNA according to claim 3, wherein the over-hang is located at the 3' terminus of a sense strand and/or an antisense strand of the interfering RNA.

5. The interfering RNA according to claim 3 or 4, wherein the over-hang is a deoxynucleoside;
preferably, the over-hang is dTdT, dTdC, or dUdU.

6. The interfering RNA according to any one of claims 1-5, comprising a sense strand and/or an antisense strand,
wherein preferably, the sense strand comprises a nucleotide sequence set forth in any one or two or more of SEQ ID NOs: 26, 28, 30, 32, 34, 36, 38, 40, 42, 44, 46, 48, 50, 52, 54, 56, 58, 60, 62, 64, 66, 68, 70, 72, and 74;
preferably, the antisense strand comprises a nucleotide sequence set forth in any one or two or more of SEQ ID NOs: 27, 29, 31, 33, 35, 37, 39, 41, 43, 45, 47, 49, 51, 53, 57, 59, 61, 63, 65, 67, 69, 71, 73, and 75.

7. The interfering RNA according to any one of claims 1-6, further comprising at least one modification, wherein the modification comprises modifications on chemical structures of a base, a sugar ring, and/or a phosphate,
wherein preferably, the modifications on a base comprise, but are not limited to, pyrimidine modifications at site 5, purine modifications at site 8, and/or 5-bromouracil substitutions;
preferably, the modifications on a sugar ring comprise, but are not limited to, substitutions of 2'-OH with H, OZ, Z, halo, SH, SZ, NH2, NHZ, NZ2, CN, or other groups, Z being an alkyl group.

8. The interfering RNA according to claim 7, wherein the modification further comprises nucleotides having inosine, queuosine, xanthine, 2'-methylribose, non-natural phosphodiester bond, and/or peptide.

9. A delivery system comprising the interfering RNA according to any one of claims 1-8, comprising the interfering RNA according to any one of claims 1-8 and a vector.

10. The delivery system according to claim 9, wherein the vector is a viral vector or a non-viral vector,
wherein preferably, the viral vector comprises: one or a combination of two or more of lentivirus vector, retrovirus vector, adenovirus vector, adeno-associated virus vector, poxvirus vector, or herpesvirus vector;
preferably, the non-viral vector comprises: any one or a combination of two or more of liposome, lipid nanoparticle, polymer, polypeptide, antibody, and aptamer.

11. The delivery system according to claim 10, wherein the lipid nanoparticle/liposome comprises: one or a combination of two or more of cationic lipid, neutral lipid, polyethylene glycol lipid, phospholipid, sterol, and anionic lipid.

12. The delivery system according to claim 11, wherein the cationic lipid comprises: one or a combination of two or more of stearamide (SA), lauryltrimethylammonium bromide, hexadecyltrimethylammonium bromide, myristyltrimethylammonium bromide, dimethyldioctadecylammonium bromide (DDAB), ((4-hydroxybutyl)azadialkyl)bis(hexane-6,1-diyl)bis(2-hexyldecanoate) (ALC-0315), 1,2-dioleoyloxy-3-(trimethylammonium)propane (DOTAP), 1,2-di-(9Z-octadecenoyl)-3-trimethylammonium-propane and 1,2-dihexadecanoyl-3-trimethylammonium-propane, 3β-[N-(N',N'-dimethylaminoethane)-carbamoyl]cholesterol (DC-cholesterol), dimethyldioctadecylammonium (DDA), 1,2-dimyristoyl-3-trimethylammonium propane (DMTAP), dipalmitoyl(C16:0)trimethylammonium propane (DPTAP), distearoyltrimethylammonium propane (DSTAP), N-[1-(2,3-diallyloxy)propyl]-N,N,N-trimethylammonium chloride (DOTMA), N,N-dioleoyl-N,N-dimethylammonium chloride (DODAC), 1,2-dioleoyl-sn-trioxy-3-ethylphosphocholine (DOEPC), 1,2-dioleoyl-3-dimethylammonium propane (DODAP), 1,2-dioleyloxy-3-dimethylaminopropane (DLinDMA), 1,2-ditetradecanoyl-3-dimethylammonium-propane, 1,2-dihexadecanoyl-3-dimethylammonium-propane and 1,2-dioctadecyl-3-dimethylammonium-propane, 1,2-dioleoyl-c-(4'-trimethylammonium)-butyryl-sn-glycerol (DOTB), dioctadecylamidoalanyl spermine, SAINT-2, polycationic lipid 2,3-dioleoyloxy-N-[2(spermine-carboxamido)ethyl]-N,N-dimethyl-1-propanaminium trifluoroacetate (DOSPA), 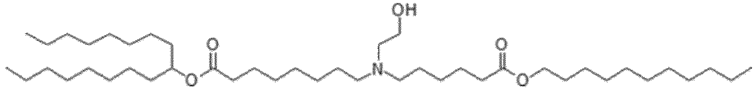 (SM-102), 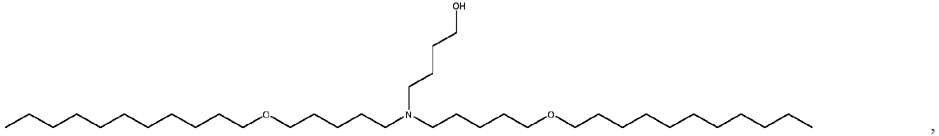 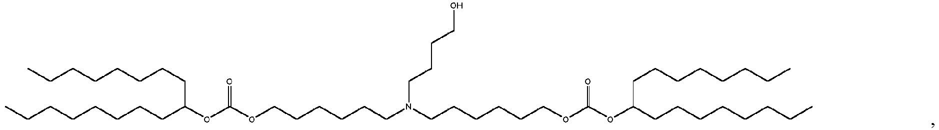 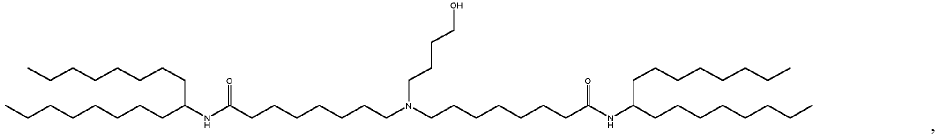 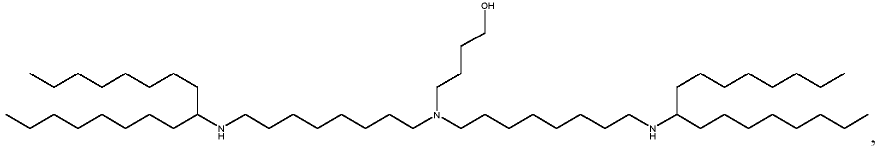 etc.

13. The delivery system according to claim 11, wherein the neutral lipid comprises: one or a combination of two or more of 1,2-distearoyl-sn-glycerol-3-phosphocholine (DSPC), 1,2-dipalmitoyl-sn-glycerol-3-phosphocholine (DPPC), 1,2-dioleoyl-sn-glycerol-3-phosphoethanolamine (DOPE), 1,2-dipalmitoyl-sn-glycerol-3-phosphoethanolamine (DPPE), 1,2-dimyristoyl-sn-glycero-3-phosphoethanolamine (DMPE), 2-dioleoyl-sn-glycero-3-phospho-(1'-rac-glycerol) (DOPG), oleoyl phosphatidylcholine (POPC), 1-palmitoyl-2-oleoyl phosphatidylethanolamine (POPE), 1,2-distearoyl-sn-glycero-3-phosphoethanolamine (DSPE), cholesterol, etc.

14. The delivery system according to claim 11, wherein the polyethylene glycol lipid comprises: one or a combination of two or more of 2-[(polyethylene glycol)-2000]-N,N-ditetradecylacetamide (ALC-0159), 1,2-dimyristoyl-sn-glyceromethoxypolyethylene glycol (PEG-DMG), 1,2-distearyl-sn-glycero-3-phosphoethanolamine-N-[amino(polyethylene glycol)] (PEG-DSPE), PEG-disterol glycerol (PEG-DSG), PEG-dipalmitoyl, PEG-dioleyl, PEG-distearyl, PEG-diacylglycerol amide (PEG-DAG), PEG-dipalmitoyl phosphatidylethanolamine (PEG-DPPE) or PEG-1,2-dimyristoylacyloxypropyl-3-amine (PEG-c-DMA), 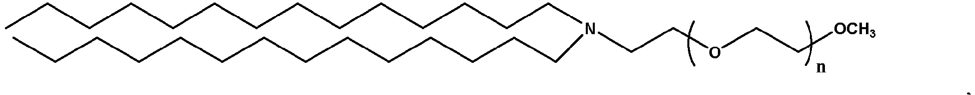 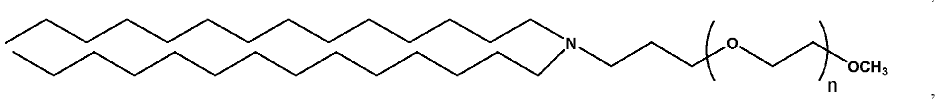 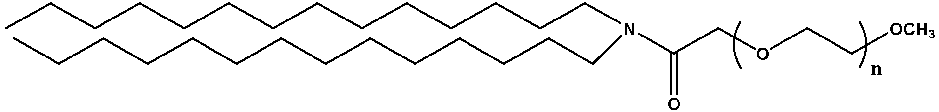 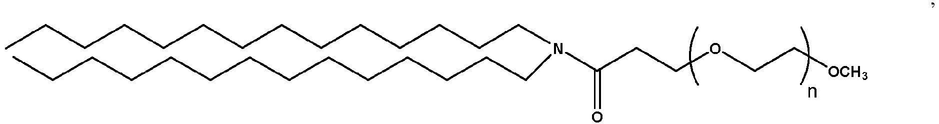 etc.,
wherein n is an integer selected from 20-300.

15. The delivery system according to claim 11, wherein the polyethylene glycol lipid is polyethylene glycol lipid with a single molecular weight, and preferably the polyethylene glycol lipid comprises: 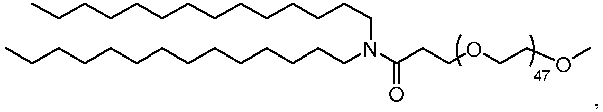 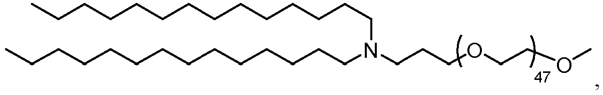 and

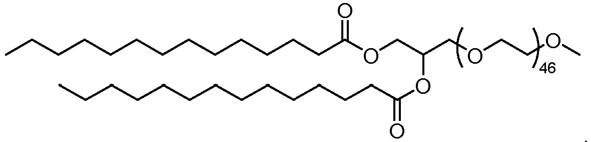

16. The delivery system according to claim 11, wherein the cationic lipid is a steroid-cationic lipid compound, wherein the structure of the compound is: 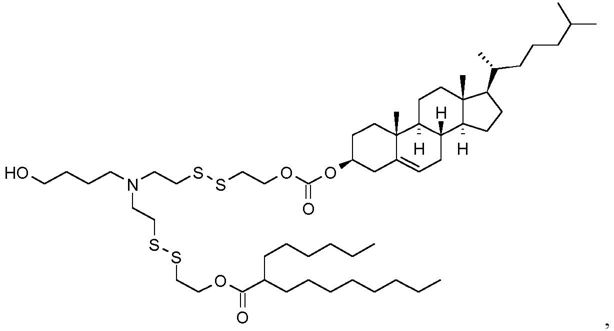 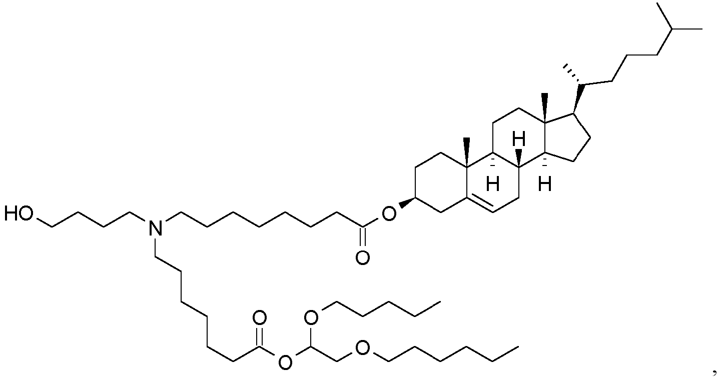 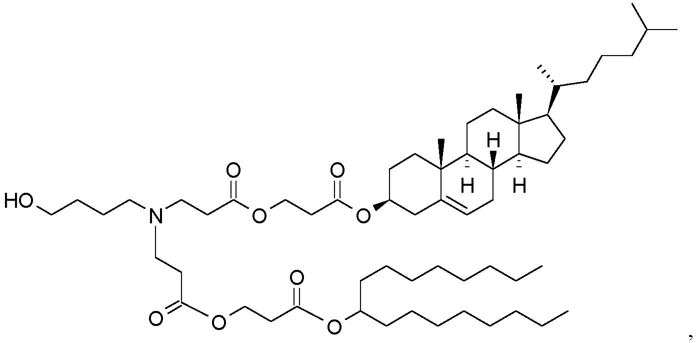 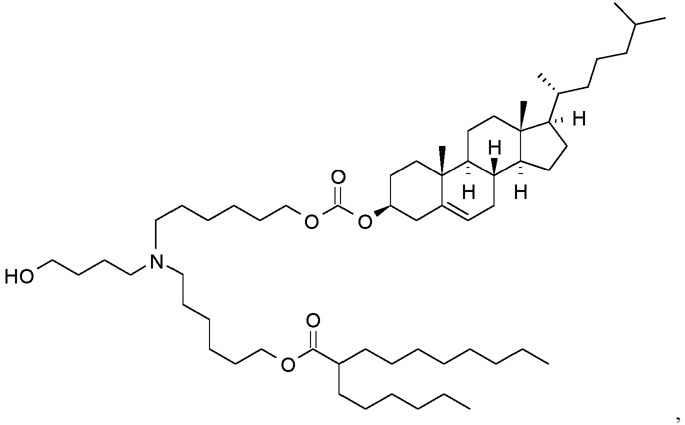 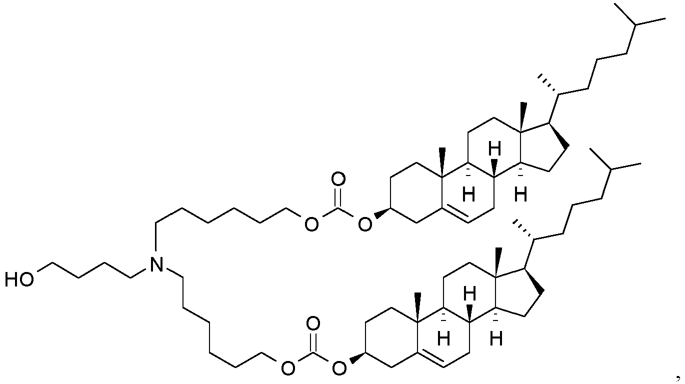 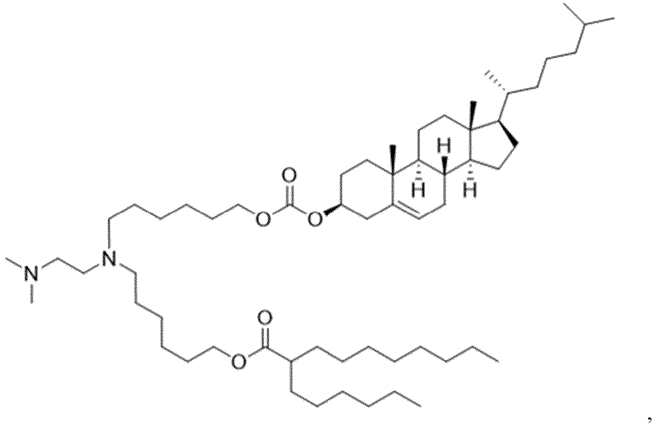 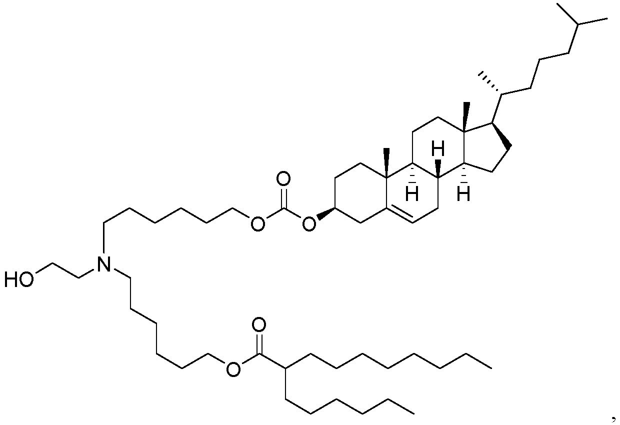 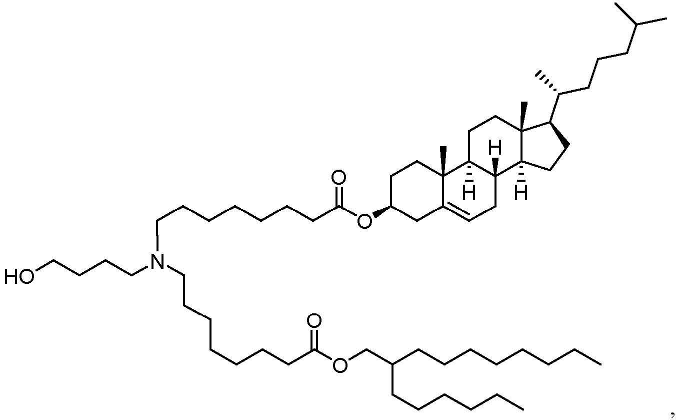 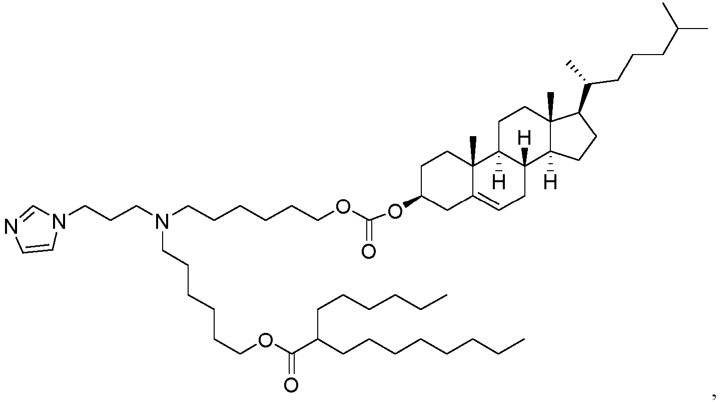 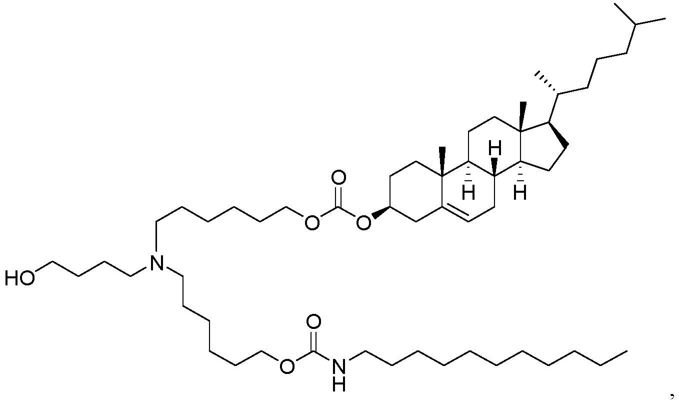 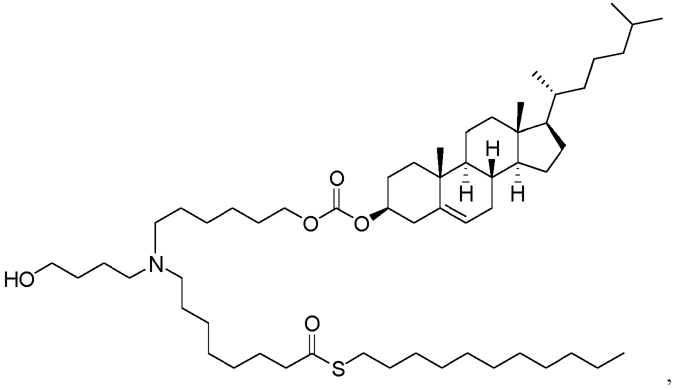 or

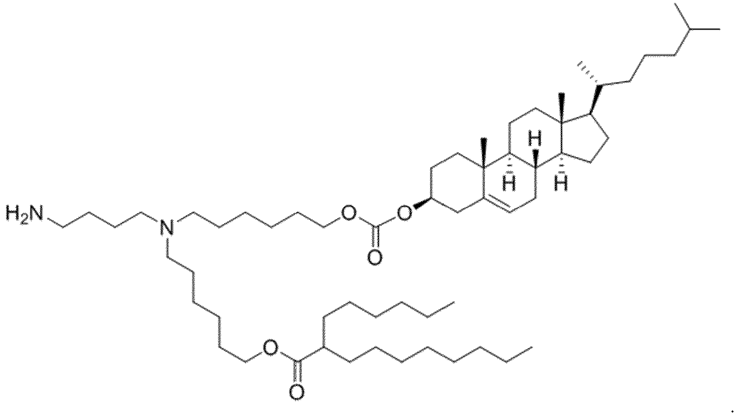

17. The delivery system according to claim 11, wherein the anionic lipid comprises: one or a combination of two or more of dioleoyl phosphatidylglycerol, dioleoyl phosphatidylethanolamine, etc.

18. The delivery system according to claim 11, wherein the sterol comprises: one or a combination of two or more of avenasterol, β-sitosterol, brassicasterol, ergocalciferol, campesterol, cholestanol, cholesterol, coprosterol, dehydrocholesterol, desmosterol, dihydroergocalciferol, dihydrocholesterol, dihydroergosterol, campesterol, epicholesterol, ergosterol, fucosterol, hexahydrolumisterol, hydroxycholesterol, lanosterol, lumisterol, saringosterol, sitostanol, sitosterol, stigmastanol, stigmasterol, cholic acid, glycocholic acid, taurocholic acid, deoxycholic acid, and lithocholic acid.

19. A cell comprising the interfering RNA according to any one of claims 1-8 or the delivery system according to any one of claims 9-18.

20. A preparation method for a cell, comprising introducing the interfering RNA according to any one of claims **1-**8 or the delivery system according to any one of claims 9-18 into cells.

21. A medicament or kit comprising the interfering RNA according to any one of claims 1-8, the delivery system according to any one of claims 9-18, or the cell according to claim 19.

22. An antibody-nucleic acid drug conjugate comprising the interfering RNA according to any one of claims 1-8 or the delivery system according to any one of claims 9-18.

23. The antibody-nucleic acid drug conjugate according to claim 22, wherein the antibody-nucleic acid drug conjugate has a general formula (I): 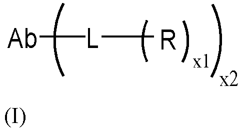
wherein R is the interfering RNA according to any one of claims 1-8;
x1 is an integer of 1-144; preferably, x1 is an integer of 1-9; more preferably, x1 is an integer of 1-3;
x2 is an integer of 1-8; preferably, x2 is an integer of 1-2;
Ab is an antibody, a protein, a polypeptide, or an oligonucleotide; and
L is a linking unit connecting Ab and R.

24. The antibody-nucleic acid drug conjugate according to claim 22, wherein the Ab is selected from a monoclonal antibody and a polyclonal antibody; preferably, Ab is a monoclonal antibody; and
more preferably, the Ab is selected from: an anti-HER2 antibody, an anti-EGFR antibody, an anti-PMSA antibody, an anti-VEGFR antibody, an anti-CD30 antibody, an anti-CD22 antibody, an anti-CD56 antibody, an anti-CD29 antibody, an anti-GPNMB antibody, an anti-CD138 antibody, an anti-CD74 antibody, an anti-ENPP3 antibody, an anti-Nectin-4 antibody, an anti-EGFRVIII antibody, an anti-SLC44A4 antibody, an anti-mesothelin antibody, an anti-ET8R antibody, an anti-CD37 antibody, an anti-CEACAM5 antibody, an anti-CD70 antibody, an anti-MUC16 antibody, an anti-CD79b antibody, an anti-MUC16 antibody, and an anti-MUC1 antibody.

25. The antibody-nucleic acid drug conjugate according to claim 23 or 24, wherein the antibody-nucleic acid drug conjugate has the following structure: 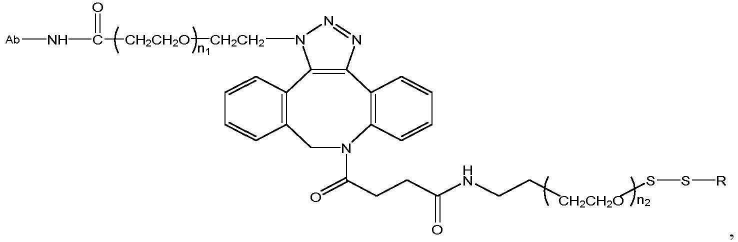 wherein n1 and n2 are independently selected from integers of 4-100, preferably integers of 4-24.

26. A pharmaceutical composition comprising the interfering RNA according to any one of claims 1-8, the delivery system according to any one of claims 9-18, or the cell according to claim 19, and a pharmaceutically acceptable excipient.

27. Use of the interfering RNA according to any one of claims 1-8, the delivery system according to any one of claims 9-18, or the cell according to claim 19 in preparing a medicament for preventing and/or treating a disease associated with B7-H3.

28. The interfering RNA according to claim 1, wherein the interfering RNA is a modified interfering RNA comprising a nucleotide sequence set forth in any one or two or more of SEQ ID NOs: 80-91.
